# EUROPEAN PATENT SPECIFICATION

(11) **EP 3 897 369 B1**
(45) Date of publication and mention of the grant of the patent: **27.03.2024**
(21) Application number: 19839774.7
(22) Date of filing: 23.12.2019
(51) Int. Cl.: A61B 5/0215

(54) **IMPLANTABLE CARDIAC SENSORS**
IMPLANTIERBARE HERZSENSOREN
CAPTEURS CARDIAQUES IMPLANTABLES

(30) Priority: 21.12.2018 US 201862783902 P; 21.12.2018 US 201862783935 P; 09.05.2019 US 201962845386 P; 30.08.2019 US 201962894260 P; 16.09.2019 US 201962901105 P
(43) Date of publication of application: 27.10.2021
(73) Proprietor: W. L. Gore & Associates, Inc., Newark, DE 19711 (US)
(72) Inventor: MINOR, David J., Newark, Delaware 19711 (US); TRAPP, Benjamin M., Newark, Delaware 19711 (US); VECCHIO, Christopher J., Newark, Delaware 19711 (US)
(74) Representative: HGF
(86) International application number: PCT/US2019/068280
(87) International publication number: WO 2020/132670

(56) References cited:
- US-A1- 2009 221 923
- US-A1- 2011 098 767
- US-A1- 2017 319 823
- US-A1- 2018 098 772

## Description

### CROSS-REFERENCE TO RELATED APPLICATION

This application claims the benefit of Provisional Application No. 62/783,902, filed December 21, 2018, Provisional Application No. 62/783,935, filed December 21, 2018, Provisional Application No. 62/845,386, filed May 9, 2019, Provisional Application No. 62/894,260, filed August 30, 2019, and Provisional Application No. 62/901,105, filed September 16, 2019.

### FIELD

The present disclosure relates to systems for measuring physiologic parameters, such as blood pressure, and more specifically to systems for measuring cardiac physiologic parameters.

### BACKGROUND

During the past decade, the number of coronary deaths in the United States has steadily decreased thanks to advancements in medical science and treatment, but the relative number of heart failure deaths has increased, indicating that more people are living with a high risk of heart failure than ever before. Generally, heart failure occurs when the heart cannot supply enough blood to the body. As a result, lower volume output leads to a higher filling pressure in the left heart to help compensate for the lack of output. Lower volume output also causes lower organ perfusion, including a reduction in kidney or renal perfusion. Reduced kidney perfusion can result in a retention of excess fluid. An acute decompensation episode is when fluid levels rise and/or vascular blood distribution declines to a state that causes the patient to experience fatigue and dyspnea (trouble breathing), thus presenting to the hospital. If left untreated, this may result in serious complications and ultimately death.

It has been observed that heart failure primarily initiates as a result of left-side heart issues. In a normal healthy heart, oxygenated blood is first carried from the pulmonary veins, through the left atrium, into the left ventricle, and into the aorta, after which the blood is carried throughout the body. Thereafter, deoxygenated blood is carried from the two vena cavae into the right atrium, through the right ventricle, and into the pulmonary arteries, which then carry the blood into the lungs for oxygenation. The pumping performance of the left ventricle can be affected by the thickening/thinning of the left ventricular wall or by the aortic/mitral valve damage, causing less blood to be pumped to the rest of the body.

There are at least two categories of heart failures: HFrEF (heart failure with reduced ejection fraction) and HFpEF (heart failure with preserved ejection fraction). In HFrEF, the left ventricle fills with enough blood, but cannot pump enough blood out due to poor contraction of the heart muscle. This is also called systolic heart failure. In HFpEF, the heart can pump blood out normally, but the left ventricle fills with less blood due to poor relaxation of the heart muscle creating less blood volume in the ventricle. This is also called diastolic heart failure. In either case, there generally is not enough blood being pumped to the body. Less commonly, biventricular failure can occur, which is when the left heart cannot pump enough blood out to the body and the right heart cannot pump enough blood to the lungs.

Pharmacological treatments are commonly employed to reduce heart pressure and prevent acute decompensation episodes. Remotely, the particular drug used is often determined by a trial and error approach using sign/symptoms such as weight gain, or by a singular intra-cardiac blood pressure measurement. Medications that are used today to reduce heart pressure and prevent acute decompensation episodes primarily include diuretics and vasodilators (nitrates, hydralazine, ace inhibitors, etc.) while other medications can be beta-blockers, inotropes, and more. Diuretics primarily target excess fluid buildup (fluid retention) and work by making the kidney release more sodium into the urine. The sodium then takes water with it from the bloodstream, thereby decreasing the amount of fluid flowing through the blood vessels and ultimately reducing intra-cardiac blood pressure. Loop diuretics, which are common in chronic heart failure, are also known to have a vasodilator effect on the venous vasculature, causing an increase in venous capacitance. Therefore, diuretics primarily help lower the preload on the heart by reducing blood volume from circulation.

Vasodilators are medications that open or dilate blood vessels, which can include nitrates, hydralazine, ace-inhibitors, and angiotensin receptor blockers, to name a few. As a result, blood flows more easily through the vessels, primarily arterial resistance vessels, and the heart does not need to pump as hard, thereby reducing intra-cardiac blood pressure. Nitrates, for example, are venous dilators at very low initial doses, but primarily increasingly affect arterial dilation in moderate to high doses (typical dosage of heart failure). Unlike diuretics, vasodilator therapy is primarily used to help reduce vascular resistance and afterload on the heart, which enhances stroke volume and cardiac output and leads to secondary decreases in left ventricular preload and venous pressures resulting in lower left sided filling pressure. Beta-blockers work to make the heart pump slower, i.e. induces lower heart rate, and with less force, thereby reducing intra-cardiac blood pressure. Inotropes work to increase the strength of ventricular contraction and therefore increase the heart rate. This medication may be used in severe cases where extremely poor perfusion exists and a ventricular assist device (VAD) or heart transplant is needed.

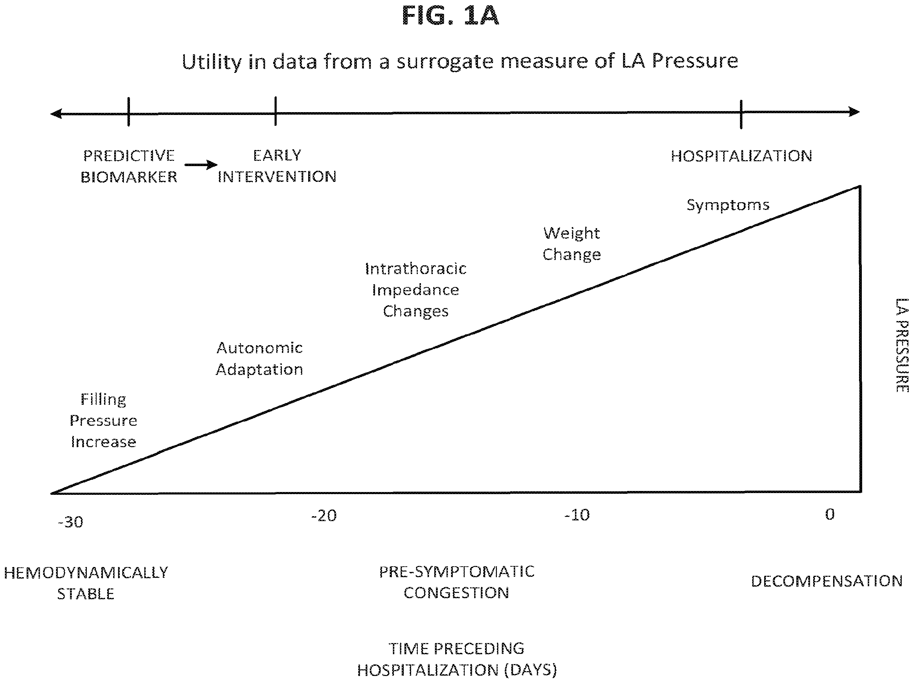

Remote pulmonary artery pressure monitoring and a corresponding medication treatment algorithm utilizing guideline medications has been proven to be effective in reducing hospitalizations due to heart failure. As shown in FIG. 1A, by monitoring the correct predictive biomarkers and performing the appropriate early interventions, the risk of hospitalization in a patient is significantly lowered. For example, in the earliest stages preceding a potential hospitalization event, measurement devices that measure an increase in the filling pressure of the heart can allow for timely treatment, resulting in a prevention of the pending hospitalization. After increased filing pressures occur, when the heart experiences pre-symptomatic congestion, the intrathoracic impedance changes. Later, other signs like a sudden weight gain, swelling in the feet and ankles, weakness or shortness of breath (dyspnea), and changes in the frequency of urination show that the body is retaining fluid. At these points, however, the congestion is typically at a later stage that is dangerously close to a decompensation episode. Therefore, it is best to treat the earliest indications because by the time later symptoms occur prior to a decompensation episode develop, it may already be too late as permanent damage may have already been done to the organs. To date, remote monitoring of cardiac filling pressure (or suitable surrogates such as Pulmonary Artery Pressure) is the only method that has led to significant reductions in hospitalizations.

To understand and treat a patient's heart failure, the hospital performs many acute analyses using various means of measurements. These include noninvasive measurements as well as invasive ones so that the medical service providers can get a better understanding of the patient's disease. Noninvasive measurements include: echocardiogram, which is used to diagnose the disease, monitor blood flow, and visualize changes in physiology; weight gain, which determines changes in fluid retention; visual inspection of the jugular vein, which determines fluid retention status; blood pressure readings, which estimate the blood flow of the body; heart rate; electrocardiography (ECG); and oxygen saturation. Invasive measurements include: right heart catheterization and left heart catheterization.

Right heart catheterization, which is performed using Swan-Ganz catheterization, can measure the central venous pressure, right atrial pressure (RAP), right ventricular diastolic and systolic pressures, pulmonary arterial diastolic and systolic pressures, and pulmonary artery wedge pressure (PAWP). Also, this method can measure the oxygen status, temperature, and heart rate of the patient, as well as calculate the cardiac output, systemic vascular resistance, and pulmonary vascular resistance. The right heart catheterization is primarily used to check pressures, cardiac output, resistance, and fluid status in the heart. Left heart catheterization can measure the left atrial pressure as well as the left ventricular diastolic and systolic pressures. The right heart catheter can be left in a patient for a few days while the medical service providers attempt to reduce the patient's intracardiac blood filling pressure back to acceptable levels using medications. This is an effective practice in an acute setting. During the ESCAPE clinical trial, the use of pressure measurements was determined as a viable means to improve a patient's overall status in the acute setting, for example by targeting a RAP of ≤ 8 mm Hg and a PAWP of ≤ 15 mm Hg. However, it was not an ongoing solution, and therefore did not prevent hospitalizations because the pressures were assumed to change relatively shortly after leaving the hospital. Therefore, a right heart catheter is primarily used to guide therapy to reduce symptoms and pressure in the acute setting.

Current diagnostic approaches can be divided into two broad settings: acute and remote. The acute setting occurs when a patient is assessed at the hospital using various methods (invasive or noninvasive). The remote setting corresponds to patient physiological parameters taken remotely, outside the hospital.

In the acute setting, a right heart catheterization may be used to give the medical service providers information for selecting appropriate medications. Generally, a right heart catheterization is viewed as useful for separating effects of volume and vascular resistance (e.g., by observing both PAWP and right atrial pressures). Medical service providers will look at the absolute values and ratios to distinguish between the two issues, particularly in the left heart failure, such that they know when fluid is offloaded and are then able to determine the status of the blood distribution. In current practice, the acute setting typically allows for more accurate measurement of the heart's health because pressure readings from different locations within the heart are taken into consideration simultaneously.

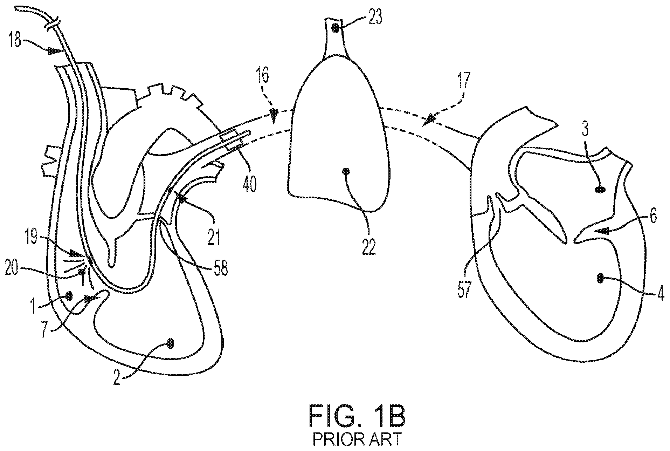

FIG. 1B is illustrative of the implementation of a right heart catheterization. The measurement device 40 is attached to the end of a pulmonary artery catheter 18 which passes through the right atrium 1, the tricuspid valve 7, the right ventricle 2, through the pulmonary valve 58, and into the pulmonary artery 16 where the device 40 takes measurement of the blood pressure as deoxygenated blood is carried into the lung 22. Then, fresh air is carried into the lung 22 from the trachea 23 after which oxygenated blood is carried through the pulmonary vein 17, the left atrium 3, the mitral valve 6, the left ventricle 4, and the aortic valve 57. The catheter 18 also has a proximal injection port which injects cold saline bolus 20 into the right atrium, and a thermistor 21 located at a distal end of the catheter to measure the temperature of the blood in the pulmonary artery 16. This method of measurement is known as thermodilution, which measures the blood flow based on the premise that when the cold saline bolus is added to the circulating blood, the rate of blood flow is inversely proportional to the rate of change in blood temperature resulting from the cold saline bolus over time. This provides a measure of cardiac output.

Pulmonary artery wedge pressure and pulmonary artery diastolic are surrogate measurements for the pressure within the left atrium and the filling pressure of the left ventricle, which is a typical area of concern in heart failure. It has been shown that the pulmonary artery and left ventricular filling pressures correlate on most occasions except for certain comorbidities such as primary pulmonary arterial hypertension. Such pressures change because of circulating volume increase (e.g. fluid retention) or declining pumping efficiency of the left ventricle (e.g., thickening, dilation, or vasoconstriction of the peripheral resistance vessels).

Various attempts have been made to remotely monitor cardiac pressures in order to identify more effective pharmacological treatment programs. These systems seek to monitor increases in intracardiac pressures to provide an early predictor of an impending acute decompensation for a patient with prior history of heart failure (e.g., as a much more reliable indicator than other measurements such as weight gain, thoracic impedance, etc.) For example, the CardioMEMS^{™} heart failure monitoring system by Abbott resides in the pulmonary artery and seeks to effectively monitor pulmonary artery pressures as a surrogate for left atrial pressure.

Other examples of remote monitoring systems include: Chronicle^{®} by Medtronic and HeartPOD^{™} by Abbott/St. Jude. A short overview of each of these systems is provided below.

With Chronicle^{®}, the measurement device resides in the right ventricle and reports an estimated pulmonary artery diastolic pressure (ePAD) to a receiving device. It has been stated that the measurements showed a correlation between right ventricular diastolic pressure, right ventricular systolic pressure, and ePAD, with the increase in all these pressure readings acting as indicators of an impending hospitalization.

HeartPOD^{™} uses a lead-and-can design with delivery of a measurement device by septal puncture method, with the measurement device remaining in the atrial septum and measuring left atrial pressure.

Another example includes the Vectorious^{™} left atrial pressure (LAP) monitoring system by Vectorious Medical Technologies which uses a pressure sensor to measure the blood pressure within the left atrium.

Over the past several decades, the development of remote systems has focused on finding a reliable predictor of upcoming hospitalization events. Measuring left sided filling pressure and surrogates have shown to be the most reliable, predictive, and effective form of remote monitoring. However, these systems show less information than acute right heart catheterization, as such systems provide limited data for accurately detecting root causes of the rise in pressure. One effect of limited data, whether in the remote or acute setting, is that medical service providers are required to utilize trial and error medication techniques for patient treatment. This remote trial and error practice can result in potential unnecessary harm to the patient, including kidney damage, further heart failure disease progression, or undetected comorbidities. For this reason, physicians are careful with their titration increases (slow increases/decreases), use creatinine lab testing as a lagging metric to detect kidney damage due to over-diuresis, and are worried about arising comorbidities (such as undetected right heart failure), and bring the patient into the office for further analysis, which may include the need for a right heart catheterization in order to determine a safe and effective treatment change.

For example, a medical service provider may first try diuretics to reduce the monitored blood pressure, if they assume that the pressure increase is due to a fluid retention issue. If this does not work, they may increase the dosage of diuretics again. If this still does not work, the medical service provider may decide that the problem is not in the fluid retention, but vascular resistance, after which an attempt may be made to use medications such as vasodilators. Lab creatinine testing may further reveal that over-diuresis (hypovolemia) led to increased damage of the kidneys. In other words, treatment methods often rely heavily on an individual medical service provider's personal experiences and intuition, which not only vary from provider-to-provider and patient-to-patient but may also extend the time needed to reliably arrive at a correct diagnosis.

In general terms, there is an ongoing need for improved physiologic measurements to assist in proper treatment regimens for patients at risk of heart failure hospitalizations. Relevant prior art is disclosed in US2009/221923, US2011/098767 and US2017319823.

### SUMMARY

Disclosed herein are medical devices, such as implantable measurement devices, for performing measurements in a heart.

According to a first aspect of the present invention there is provided a medical system for determining a treatment regimen for a patient with a condition, the system comprising:
at least one sensor configured to sense right atrial pressure and left atrial pressure; and
a receiver configured to receive measurement data corresponding to the sensed right atrial pressure and the sensed left atrial pressure, to receive the condition of the patient, and output to a display device the received measurement data:
   a memory unit configured to store the received measurement data and the condition of the patient; and
   a processor configured to: compare the sensed right atrial pressure to a baseline right atrial pressure to determine a first comparison;
   compare the sensed left atrial pressure to a baseline left atrial pressure to determine a second comparison; and
   determine the treatment regimen for the patient based on the comparisons and the condition of the patient, wherein the condition is at least one selected from the group of: left heart failure, right heart failure, and primary pulmonary disorder.

According to a further aspect of the present invention there is provided a computer-implemented method for determining a treatment regimen for a patient with a condition, the method comprising:
receiving at least one measurement corresponding to a sensed right atrial pressure for the patient; receiving at least one measurement corresponding to a sensed left atrial pressure for the patient; $
receiving the condition of the patient; comparing the sensed right atrial pressure to a baseline right atrial pressure to determine a first comparison;
comparing the sensed left atrial pressure to a baseline left atrial pressure to determine a second comparison;
determining the treatment regimen for the patient based on the first comparison, the second comparison, and the condition of the patient; and $
outputting to a display device: the at least one measurement corresponding to the sensed right atrial pressure, the at least one measurement corresponding to the sensed left atrial pressure, and the determined treatment regimen,
wherein the condition of the patient is at least one selected from the group of: left heart failure, right heart failure, and primary pulmonary disorder.

The foregoing Examples are just that and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1A is a graph showing the utility in data from a surrogate measurement of left atrial pressure in reducing hospitalizations due to heart failure;
FIG. 1B is a schematic diagram of a heart and a lung of a patient using the prior-art measurement device (Swan Ganz right heart catheter) as discussed herein;
FIG. 2 is a cross-sectional diagram of a heart which uses a measurement device according to some embodiments;
FIG. 3 is a cross-sectional diagram of a heart which uses a measurement device according to some embodiments;
FIG. 4 is a cross-sectional diagram of the commissures of a tricuspid valve;
FIG. 5 is a cross-sectional diagram of the commissures of a mitral valve;
FIG. 6 is a schematic diagram of a heart and a lung of a patient using a measurement device according to some embodiments;
FIG. 7 is a schematic diagram of a heart and a lung of a patient using another measurement device according to some embodiments;
FIG. 8 is a close-up view of a measurement device according to some embodiments;
FIG. 9 is a cross-sectional diagram of the content of right atrium electronics in the measurement device of FIG. 8;
FIG. 10 is a cross-sectional diagram of the content of left atrium electronics in the measurement device of FIG. 8;
FIG. 11 is a close-up view of a measurement device according to some embodiments;
FIG. 12 is a cross-sectional diagram of two examples of the contents of right atrium electronics in the measurement device of FIG. 11;
FIG. 13 is a cross-sectional diagram of the contents of left atrium electronics in the measurement device of FIG. 11;
FIG. 14 is a cross-sectional diagram of a heart which uses a measurement device according to some embodiments, where a portion of the measurement device is punctured by a trans-septal needle;
FIG. 15 is a close-up view of the measurement device of FIG. 14 being punctured;
FIG. 16 is a close-up view of another measurement device according to some embodiments, where a portion of the measurement device is punctured by a trans-septal needle;
FIG. 17 is a close-up view of a mesh configuration used in a measurement device according to some embodiments;
FIG. 18 is a schematic diagram of a wearable harness for an external reader device for a measurement device according to some embodiments;
FIG. 19 is a schematic diagram of a syringe or catheter with a needle containing a sensor tethered to a wire according to some embodiments;
FIG. 20 is a schematic diagram of a sensor connected to a subcutaneous implant according to some embodiments;
FIG. 21 is a cross-sectional view of a heart with the sensor implanted and immobilized using a pledget according to some embodiments;
FIG. 22 illustrates one example of a location of the subcutaneous implant in the patient's body according to some embodiments;
FIG. 23 illustrates a block diagram of a method to determine actions that need to be taken based on pressure measurements according to some embodiments;
FIG. 24 illustrates a medication administration reference table using two sets of measurement data as implemented by the method in FIG. 23;
FIG. 25 is a cross-sectional diagram of a measurement device according to some embodiments;
FIG. 26 is a cross-sectional diagram of a measurement device according to some embodiments;
FIG. 27 is a cross-sectional diagram of a measurement device according to some embodiments;
FIG. 28 is a cross-sectional diagram of a measurement device according to some embodiments;
FIG. 29 is a cross-sectional diagram of a measurement device according to some embodiments;
FIG. 30 is a cross-sectional diagram of a measurement device according to some embodiments;
FIG. 31 is a side view of a measurement device according to some embodiments;
FIG. 32 is a cross-sectional diagram of the measurement device of FIG. 31;
FIG. 33 is a side view of the sensing element of FIG. 31 according to some embodiments;
FIG. 34 is a schematic diagram of an electronics housing component according to some embodiments.
FIG. 35 illustrates a flow diagram of a method for determining a treatment regimen for a patient according to some embodiments;
FIG. 36 illustrates an exemplary diagnostic table and an exemplary treatment regimen table referenced by the method in FIG. 35 for a patient diagnosed with left heart failure;
FIG. 37 illustrates an exemplary diagnostic table and an exemplary treatment regimen table referenced by the method in FIG. 35 for a patient diagnosed with right heart failure;
FIG. 38 illustrates an exemplary diagnostic table and an exemplary treatment regimen table referenced by the method in FIG. 35 for a patient diagnosed with primary pulmonary disorder;
FIG. 39 illustrates an exemplary diagnostic table and an exemplary treatment regimen table referenced by the method in FIG. 35 for a patient diagnosed with left heart failure and right heart failure;
FIG. 40 illustrates an exemplary diagnostic table and an exemplary treatment regimen table referenced by the method in FIG. 35 for a patient diagnosed with left heart failure and primary pulmonary disorder;
FIG. 41 illustrates an exemplary diagnostic table and an exemplary treatment regimen table referenced by the method in FIG. 35 for a patient diagnosed with right heart failure and primary pulmonary disorder; and
FIG. 42 illustrates an exemplary diagnostic table and an exemplary treatment regimen table referenced by the method in FIG. 35 for a patient diagnosed with left heart failure, right heart failure, and primary pulmonary disorder.

### DETAILED DESCRIPTION

This disclosure is not meant to be read in a restrictive manner. For example, the terminology used in the application should be read broadly in the context of the meaning those in the field would attribute such terminology.

As the terms are used herein with respect to ranges of measurements "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error, differences in measurement and/or manufacturing equipment calibration, human error in reading and/or setting measurements, adjustments made to optimize performance and/or structural parameters in view of differences in measurements associated with other components, particular implementation scenarios, imprecise adjustment and/or manipulation of objects by a person or machine, and/or the like.

Certain terminology is used herein for convenience only. For example, words such as "top", "bottom", "upper," "lower," "left," "right," "horizontal," "vertical," "upward," and "downward" merely describe the configuration shown in the figures or the orientation of a part in the installed position. Indeed, the referenced components may be oriented in any direction. Similarly, throughout this disclosure, where a process or method is shown or described, the method may be performed in any order or simultaneously, unless it is clear from the context that the method depends on certain actions being performed first.

Various embodiments are directed toward implantable medical devices such as device for performing physiologic measurements in the left and right sides of the heart. In certain instances, the various aspects of the present disclosure relate to methods and devices for performing pressure measurements. Additionally, the present disclosure also includes a medical treatment system for determining administration of medications to a patient based on the measurements performed.

Various examples relate to systems and methods for directly taking left atrial and/or ventricular measurements (e.g., blood pressure). The left side of the heart takes oxygenated blood from the lungs and distributes it to the rest of the body, while the right side of the heart carries deoxygenated blood from the body to the lungs. Various examples relate to sensor designs that avoid clots (emboli) and other unwanted side effects of placing a sensor in the heart. When a foreign object such as a sensor is implanted within the heart, a blood clot may form on the surface of the implanted foreign object, which may break off and form an embolus. The damage done by an embolus varies depending on its location. If the implanted, foreign object is in the right side of the heart, the embolus would likely travel to the lungs, but if the implanted foreign object is in the left side, an associated embolus could travel to any part of the body, including an artery leading to the brain causing thrombotic stroke. In addition to effective, remote setting left side measurements, various examples also relate to measuring in vivo conditions in two different portions of the heart (e.g., instead of only measuring a single region).

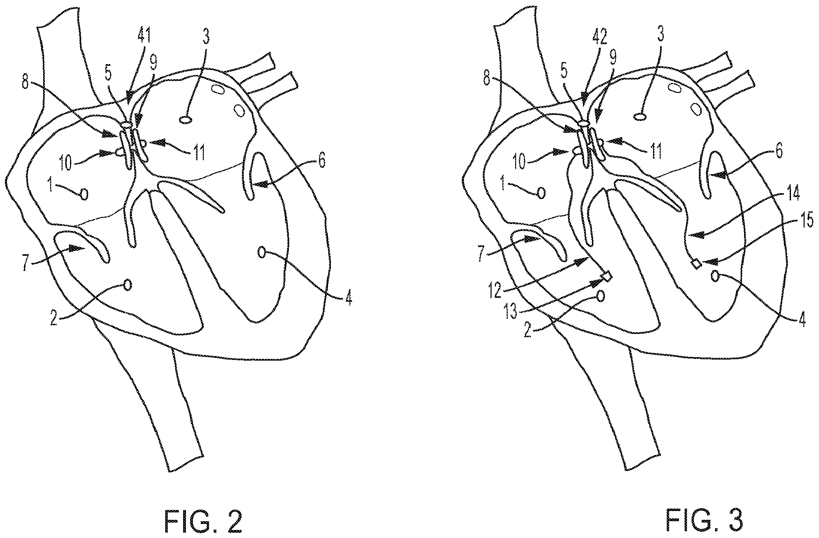

FIG. 2 shows an embodiment of a measurement device 41 according to the present disclosure. The measurement device has a right-side sensing element 10 and a left side sensing element 11, both of which sense and measure the pressure level within the respective side in which they are integrated. For example, in this example, the right-side sensing element 10 measures the pressure level in the right atrium 1, while the left side sensing element 11 measures the pressure level in the left atrium 3 of the patient's heart. The pressure sensing elements 10,11, may incorporate MEMS technology such as but not limited to capacitive or piezoresistive MEMs sensors or other pressure measurement means, as suitable, to measure intracardiac pressure levels.

As shown, the measurement device 41 has a right anchoring disc 8 and a left anchoring disc 9 which work together to help hold the measurement device 41 in place. As shown in the figure, the two discs 8,9 are designed to sandwich the atrial septum 5 between the two atria 1,3 (e.g., either actively engaging or contacting each side in an opposing manner). The placement of the measurement device 41 can be achieved with a catheter procedure and septal puncture. The sensing elements 10, 11 may be utilized along with a variety of devices that anchor to and extend across the atrial septum. Suitable examples may be found in a variety of Applicant's patent disclosures, including US9949728, "Septal closure device with centering mechanism"; US20170042705 "Implantable Product with Improved Aqueous Interface Characteristics and Method for Making and Using the Same"; US9861346 "Patent foramen ovale (PFO) closure device with linearly elongating petals"; US9636094 "Sealing device and delivery system"; and US20170105711, "Sealing Device and Delivery System."

In the example of FIG. 2, the measurement device 41 leaves no hole after surgery because of the anchoring discs 8,9 acting as occluders. The measurement device 41 can be configured to promote tissue ingrowth (e.g., into the anchoring discs 8,9) for any of a variety of reasons, including better tissue integration, reduced erosion, reduced thrombosis, or other beneficial features. Reduction of thrombosis can be particularly important on the left side of the heart. In some configurations, the measurement device 41 is configured such that the left side sensing element 11 is relatively low profile (e.g., relatively flat in profile). A relatively low profile may assist with reducing the potential for thrombosis. In some examples, some level of tissue overgrowth over the sensor may also be permitted while still permitting proper functioning of the left side sensing element 11. For example, the pressure on the left side (e.g., left atrium) may be read through a relatively thin layer of tissue, if necessary.

FIG. 3 shows another embodiment of a measurement device 42 according to the present disclosure. In addition to the anchoring discs 8,9 and sensing elements 10,11 shown in FIG. 2, there are also sensing tethers 12,14 extending into the respective ventricles. Specifically, a right ventricle sensing tether 12 extends from the right side of the measurement device 42 (e.g., from the right-side sensing element 10), into the right ventricle. A remote right ventricle sensing element 13 may be attached to the right ventricle wall (e.g., using soft tissue anchors and / or tissue ingrowth features). The remote right ventricle sensing element 13 is configured to measure the pressure inside the right ventricle. The remote sensing element includes a MEMs sensor, for example a piezoresistive embodiment, but does not necessarily include the associated electronics and instead has wire leads providing the direct signal transmission back to the electronics housing.

Similarly, a left ventricle sensing tether 14 extends from the left side of the measurement device 42 (e.g., from the left side sensing element 11) into the left ventricle. A remote left ventricle sensing element 15 may be attached to the left ventricle wall (e.g., using soft tissue anchors and / or tissue ingrowth features). The remote left ventricle sensing element 15 is configured to measure the pressure inside the left ventricle.

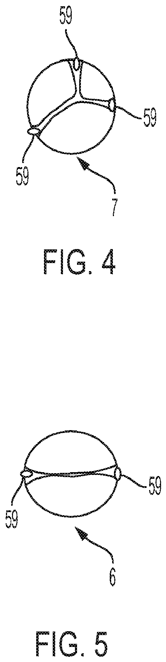

The remote sensing elements 13,15 are configured to measure pressures in different portions of the heart than the sensing elements 10,11. In at least this manner, the measurement device 42 is configured to provide additional measurement data (e.g., left ventricular and right ventricular pressure data) for analysis. The sensing tethers 12, 14 may be arranged or otherwise positioned to extend through the commissures of the valves that reside between the right atrium and the right ventricle (tricuspid valve) and the left atrium and left ventricle (mitral valve). FIGs. 4 and 5 show optional positions for the sensing tethers 12, 14, which include positions adjacent the commissures 59 located between the leaflets of the tricuspid valve 7 (FIG. 4) or the mitral valve 6 (FIG. 5) through which the sensing tethers 12,14 extend to reach their respective ventricles. In various examples, by positioning the tethers 12, 14 adjacent the commissures 59 the impact of the tethers on valve function and / or likelihood of thrombosis may be reduced.

In one example, additional sensors may be incorporated into the sensing tethers 12,14. In another example, the additional sensors may be implemented into other elements of the measurement device 42 at the points of tether attachment to measure a force, i.e. tensile stress, on the tethers 12,14. The force on the tethers 12,14 may be used as an indication of local blood flow velocity within the heart, and this measurement data may be used by itself or in combination with other measurement parameters to assess cardiac function of the patient. Advantages of measuring such force on the tethers 12,14 including the ability to obtain data which serves as indicators relating to the cardiac function such as mitral inflow velocity, tricuspid flow, and severity of a potential regurgitation, for example, which may be difficult to detect using other means of measurement. To effectively measure this force, the tethers 12,14 and remote sensing elements 13,15 in this example are at least partially free-floating (i.e. not attached to the walls of an atrium or ventricle). In addition to, or as an alternative to measuring pressure and/or force, the various sensing elements may be configured to measure temperature (e.g., by including one or more thermistor elements). By including temperature sensing capabilities, a cold bolus (e.g., fluid) may be introduced into the cardiac system and the rate of temperature equalization may be used to determine cardiac output at various locations in the heart. In contrast to methods that utilize a cold fluid bolus, various examples include use of a cold air bolus in the lungs to measure the rate at which temperature equalizes from blood returning from the lungs.

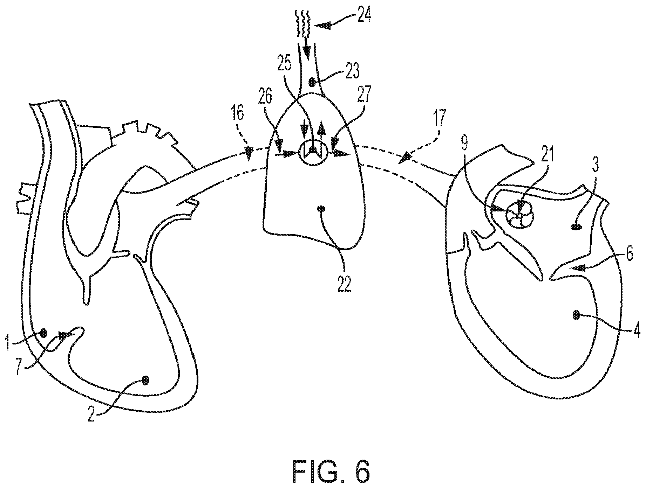

FIG. 6 shows how a cardiac output is affected by and therefore can be measured via thermodilution with a cold air bolus and a left atrium sensor, or thermistor 21, according to some embodiments. Initially, cold air bolus 24 is injected into the lung 22 (e.g., by introducing inhalation of a bolus of cold air into the lungs through the patient's trachea 23). Then, heat exchange 25 takes place when warmer deoxygenated blood 26 enters the lung through the pulmonary artery 16 and is then cooled by the cold air bolus 24 during oxygenation. Afterwards, cooler oxygenated blood 27 leaves the lung through the pulmonary vein 17 into the left atrium 3 where the thermistor 21, immobilized using the left anchoring disc 9, measures the temperature in the left atrium 3. If more blood is flowing, the temperature within the left atrium 3 would return to normal temperature sooner than when less blood is flowing. As such, the inhalation of cold air in this case can be used to determine the initial drop in the blood temperature in the left atrium 3, which is used to correlate the time for the temperature to return to normal. The rate at which the temperature returns to normal correlates with the cardiac output.

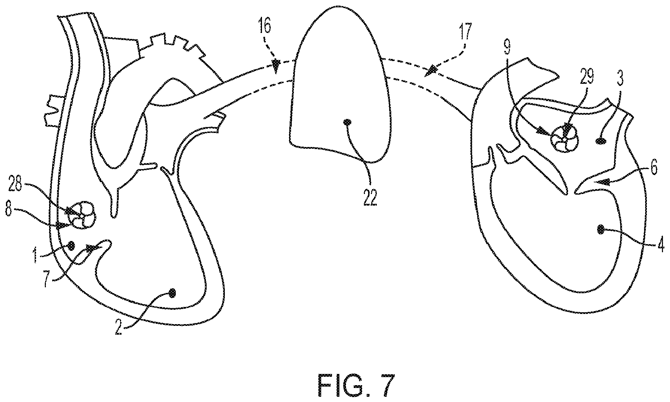

As a further additional or alternative feature, one or more O2 sensors may be included at one or more of the first pair of sensing elements 10,11 and the remote second pair of sensing elements 13,15. FIG. 7 shows a diagram of blood flow from the right to left side of the heart through a lung and can be used to describe how cardiac output can be measured via O2 blood saturation sensors 28,29 (e.g., using Fick's Law). According to various examples, a right O2 saturation sensor 28 is placed in the right atrium 1 (e.g., associated with the sensing element 10 immobilized using the right anchoring disc 8), and a left O2 saturation sensor 29 is placed in the left atrium 3 (e.g., associated with the sensing element 11 immobilized using the left anchoring disc 9), to measure the SvO2 and SaO2 levels in their respective areas of measurement.

Fick's Law dictates that the blood flow to the patient's heart can be calculated using a marker substance, which in this case is oxygen (O2). The necessary data for making such calculations include the amount of oxygen taken up by the heart per unit time, the O2 blood saturation in the pulmonary artery, and the O2 blood saturation in the pulmonary vein. In this case, the O2 blood saturation of the pulmonary artery 16 is measured at the right atrium 1, and the O2 blood saturation of the pulmonary vein 17 is measured at the left atrium 3. Other data for the calculation can include maximal oxygen uptake (VO2 max), which is the maximum rate of oxygen consumption measured during incremental exercise, and hemoglobin test, which in combination with the arterial and venous percentages will determine oxygen concentration.

As explained above, different sensors can be implemented in the embodiments as disclosed herein (e.g., pressure, flow, temperature, and/or O2), with each measurement contributing vital data regarding the health of the patient's heart. The sensors themselves can be of various shapes and sizes, as deemed suitable by a person of ordinary skills in the art, to be implemented inside a patient's heart.

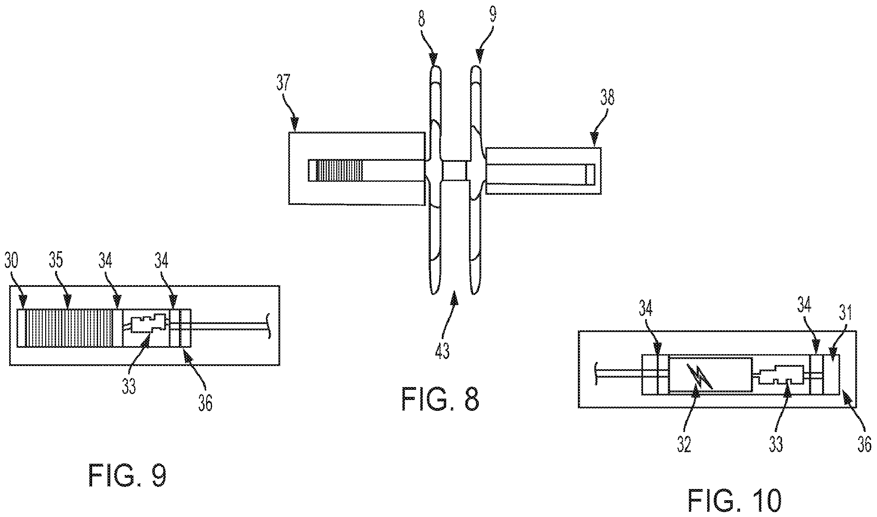

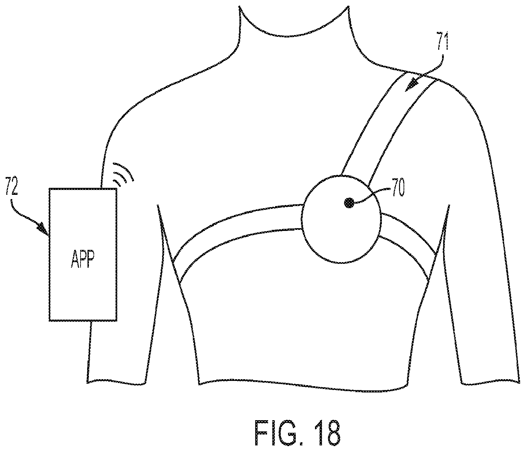

FIGs. 8-10 show additional details of possible sensor element configurations. As shown in FIG. 8, a measurement device 43 has a right atrium sensing element 30 included in right atrium electronics 37 and a left atrium sensing element 31 included in left atrium electronics 38. The electronics 37,38 are attached to their respective anchoring discs 8,9 as shown in FIG. 8. FIG. 9 shows that the electronics 37,38 both include a control module 33 (e.g., printed circuit board) in a proximal part 34 of a case 36 with regard to the anchoring discs 8,9. The case 36 may be made of titanium, stainless steel, or other suitable materials. The right atrium electronics 37 further include the right atrium sensing element 30 and an antenna coil 35. The left atrium electronics 38 further include the left atrium sensing element 31 and a power source 32 (e.g., battery and/or wireless power source). In this configuration, an external reader device, such as the external charger and communications relay 70 in FIG. 18, charges the power source 32 as well as communicating with the electronics 37,38 to obtain the measurement data stored in a memory implemented in the control modules 33.

The control modules 33 can be designed such that they are configured to perform a sequence of steps for taking measurements within different portions of the heart (e.g., each of the chambers), whether the measurements are in blood pressure, temperature, and/or oxygen saturation), as well as to store the data until the external reader device can access the data, usually wirelessly. Furthermore, the power source 32 can be any suitable power source that can be used in this implementation. For example, the power source can be coupled to a charging coil which enables inductive charging of the power source such that the external reader device can remotely charge the power source from outside the patient's body, which reduces the need to exchange the power source once it runs out of power. For example, the antenna coil 35 can be used as a charging coil in addition to performing relay/communication functions.

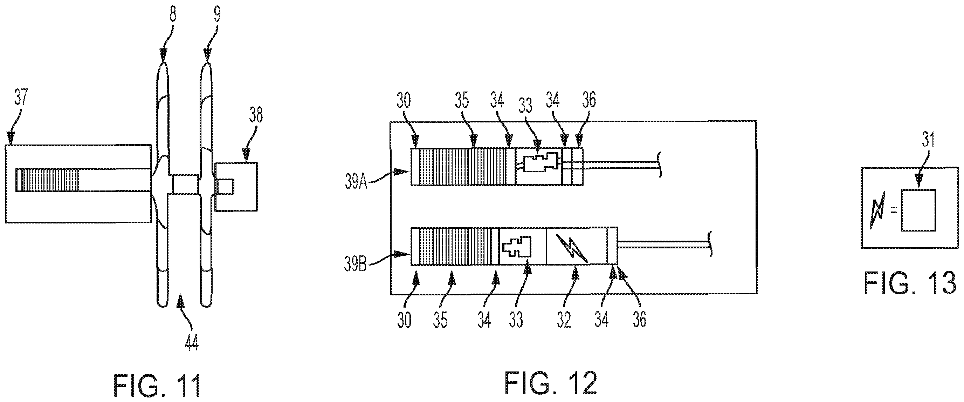

FIGs. 11-13 show various configurations that may be used in various examples. As shown, the left atrium electronics 38 of a measurement device 44 only include the left atrium sensing element 31 (e.g., sensing, temperature and/or O2). Minimizing the number of components / elements on the left side of the device may help minimize the overall size of the device and the associated amount of foreign material in the left atrium. As described above, this may reduce the potential for thrombosis and foreign body response. As such, in various examples (e.g., as shown in inset view of the configuration 39B), the right atrium electronics 37 include the power source 32 to power the sensing elements 31, any associated data storage elements, and more generally the control module 33. In another example as shown in configuration 39A of the inset view of FIG. 12, the right atrium electronics 37 do not include an onboard power source. In such embodiments, measurement data may only be taken when an external reader device is engaged to power the measurement device 44 (e.g., using inductive power to activate the sensing elements 30,31 and control module 33).

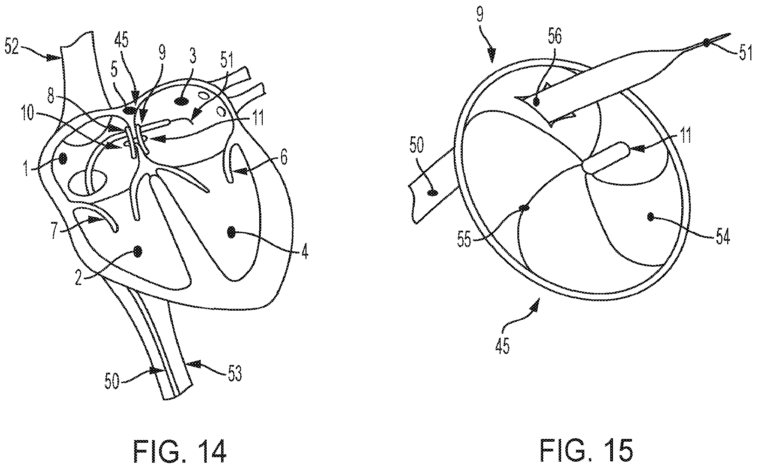

In various examples, one or more of the measurement devices 41,42,43,44 (e.g., one or both of the anchoring discs 8,9) is configured to be fenestrated, or crossed by a surgical implement (e.g., trans-septal needle) following implantation. FIGs. 14 and 15 show a puncture needle 51 attached to the end of a catheter sheath 50 penetrating the surfaces of the anchoring discs 8,9 to indicate that the measurement device 45 is re-crossable (e.g., may be penetrable by a needle and followed by a sheath).

During certain procedures or operations, it may be imperative to enter the left atrium 3 even though the measurement device 45 has been implemented in the atrial septum 5 between the atria 1,3. In such case, the measurement device 45 is configured to have re-crossable surfaces in the anchoring discs 8,9 so that the puncture needle 51 can penetrate these surfaces to perform the procedures. FIG. 14 shows the needle 51 and catheter sheath 50 entering the right atrium 1 from the inferior vena cava 53, but in some instances may enter the right atrium 1 from the superior vena cava 52 as necessary. In this embodiment, the surface of the anchoring discs 8,9 includes a material than can be safely penetrated, such as an expanded polytetrafluoroethylene (ePTFE) membrane 54, although other suitable materials can be implemented to provide re-crossing capability. The outer edge of the anchoring disc 9 is defined by a nitinol frame 55 which also extends radially from the left side sensing element 11 to the outer edge, although other suitable materials may be used for the frame as well. The needle 51 forms a puncture hole 56 in the membrane 54 to allow for the catheter sheath 50 to pass through.

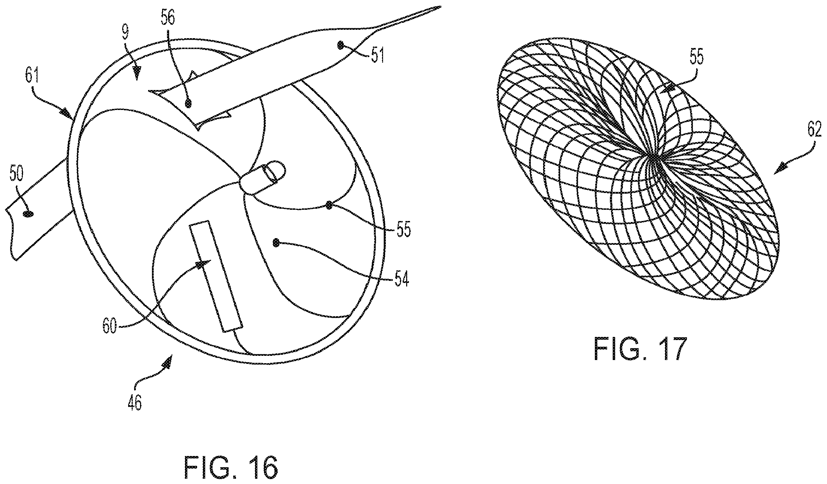

FIG. 16 shows another embodiment as disclosed herein where the left side sensing element 60 of a measurement device 46 is located on a portion of the left anchoring disc 9. For example, the left side sensing element 60 may be located on membrane material (e.g., ePTFE membrane 54) of the left anchoring disc 9, as opposed to the frame of the left anchoring disc 9 (e.g., center eyelet or outer frame). In this embodiment, the left side sensing element 60 is coupled to the membrane 54 to help prevent the sensing element 60 from protruding outward from the septal wall or reduce the amount the left sensing element 60 protrudes from the septal wall from the measurement device 46. In this embodiment, an antenna coil 61 is implemented in the anchoring disc 9. As shown, the sensing element 60 is attached to the antenna coil 61, and the antenna coil 61 is wrapped around the ePTFE membrane 54 to form the outer edge of the left anchoring disc 9, thereby defining the periphery of the left anchoring disc 9. Additional embodiments may further reduce the amount the left sensing element 60 protrudes from the septal wall. For example, in one embodiment, the left anchoring disc 9 may be replaced with a small tissue anchoring structure which help align the outer surface of the left sensing element 60 to be substantially flush with the surface of the surrounding septal wall, such that the sensing element 60 would not visibly protrude from the septal wall. For example, small hooks or other suitable structures may be implemented to hold the left sensing element 60 in place. In another embodiment, a cover or other similar component may be employed over the left sensing element 60 to prevent the sensing element from substantially protruding into the left atrium. The cover may be made of a material that is chemically inert such as low temperature isotropic (LTI) carbon and diamond like carbon (DLC), or polymers such as polytetrafluoroethylene (PTFE), expanded PTFE, or polyethylene terephthalate (PET). In some examples, the cover may be a thin film placed over the left sensing element 60 to promote tissue ingrowth over the sensing element.

FIG. 17 shows another potential configuration for the medical device 46 where the left anchoring disc 62 uses a frame (e.g., nitinol) configured into a mesh design that defines the left and/or right disc(s). The configuration of FIG. 17 may or may not employ a cover or membrane 54 (e.g., ePTFE). The re-crossable surface is designed such that catheters with a French gauge suitable to fit between the frame elements (e.g., up to 24 Fr) may fit through the anchoring disc(s) without interfering with the electronics. Also, it should be appreciated that although the anchoring discs illustrated in the figures are relatively flat and circular in structure, any of the anchoring disc configurations described herein can use other shapes (e.g., rectangular, triangular, etc.) as well. Moreover, the anchoring discs may be configured with a curved side profile (e.g., concave and / or convex) to accommodate for the different contours defining the surface within the heart to which the anchoring discs are to be engaged.

In one embodiment, the measurement device 45,46 can also act as a therapeutic device, such as an intra-atrial shunt, a controllable intra-atrial shunt, an occluder for atrial septal defects (ADS), and so on. The measurement device 45,46 can act as such a controllable shunt because it is located at an interatrial septum between the left and right atria, and the membrane 54 can be opened via interventional or noninvasive procedures. As such, the membrane 54 may be expanded, contracted, opened, closed, fenestrated, sealed, punctured, resealed, traversed, or crossed using appropriate tools during different procedures to actuate the controllable shunt. The use of a needle to make a puncture hole 56 in the membrane 54 as discussed above is one example of the interventional procedure. Other interventional procedures include mechanical, thermal, laser, ultrasound, and inductive methods. On the other hand, the opening of the hole can be triggered via wireless, extracorporeal energization, including inductive energy transfer and ultrasound energy transfer. In one embodiment, the membrane 54 can be melted to form an opening after exposing the membrane 54 to thermal or ultrasound energy, i.e. via thermal activation. An advantage in having an opening in the membrane 54 includes, when the measurement device 45,46 is located between the left and right atria, reducing the left atrial pressure when it rises to a life-threatening level. One advantage in this configuration is that even after the opening is formed, the measurement device 45,46 can continue taking measurements within the two atria. The size of the shunt can be adjusted based on the required degree of pressure relief. For example, if the pressure is significantly higher than the normal level such that the pressure must be lowered immediately, the shunt can be opened wider. When the shunt is opened via mechanical piercing or thermal ablation, it can prevent embolization as well. Furthermore, a pressure-sensitive valve may be implemented in the measurement device 45,46 such that the membrane 54 opens to form the shunt above a threshold pressure level. In another example, the valve may also track and transmit its status (i.e. whether the shunt is open or closed in the valve, as well as the degree of opening in the shunt) which may serve as an indication of a pressure differential within the heart. Therefore, a remote monitoring system (for example a remote device 72 in FIG. 18) which receives data from the valve can use the status data of the valve to determine a difference in pressure between the left and right atria.

FIG. 18 shows an example of external charging and communications relay according to some examples. As shown, the external charger and communications relay 70 is a device which can charge or power a power source of the measurement device (for example, the battery 32 in the measurement devices 43,44) via electromagnetic induction, as well as to communicate with the measurement device 43 or 44 to obtain the measurement data. In one example, the external charger communications relay 70 is a device which inductively couples with the measurement devices 43,44 to directly power the measurement devices 43,44 such that an on-board power source, for example a battery implemented within the measurement devices 43,44, is not required. In one example, the external charger communications relay 70 wirelessly powers the measurement devices 43,44 via radiofrequency (RF) electromagnetic radiation. The external charger and communications relay 70 may be worn (e.g., using a harness 71) such that the location of the charger and relay 70 is placed at an operable location for the charger and relay 70 to charge and obtain data from the measurement device. A monitoring system 72, which can be a smart device such as a smartphone, can be used by the patient or other party (e.g., medical service provider or remote monitoring facility) to receive information regarding the measurement data via an application software in the monitoring system. For example, the remote device 72 can visually show the blood pressure, temperature, and/or oxygen saturation in a simple, user-friendly interface. If the patient is visually impaired or prefers audio notifications, the remote device 72 can provide audio output to alert the patient if the sensor measurements indicate that the patient's heart may be at risk of acute decompensation episodes, so that the patient can go to a hospital for a further examination. The remote device 72 can also upload the measurement data onto a server (not shown) to be collected by medical service providers or a database to remotely monitor the conditions of the patient's heart.

Based at least upon the foregoing, it should be appreciated that a variety of sensor locations are contemplated and may be implemented in any combination.

For example, to measure the left ventricular pressure, a tethered sensor can be sent off the left anchoring disc, between the mitral valve leaflets, and into the left ventricle, where tissue ingrowth can implement the sensor into the wall of the left ventricle. The sensor directly measures the left ventricular systolic and diastolic pressure, which also gives a direct indication of systolic systemic blood pressure.

To measure the aortic pressure, the tethered sensor can be sent off the left anchoring disc, between the mitral valve leaflets, through the aortic valve, and into the aorta, where the sensor is secured to the wall of the aorta. This placement allows for direct measurement of the aortic pressure which gives a direct indication of systolic and diastolic blood pressures.

To measure the right ventricular pressure, the tethered sensor can be sent off the right anchoring disc, between the tricuspid valve leaflets, and into the right ventricle, where tissue ingrowth can implement the sensor into the wall of the right ventricle. The sensor directly measures the right ventricular pressure which gives a direct indication of systolic and diastolic right ventricular pressures.

To measure the pulmonary artery pressure, the tethered sensor can be sent off the right anchoring disc, between the tricuspid valve leaflets, through the pulmonary valve, and into the pulmonary artery, where it is secured. This placement allows for direct measurement of the pulmonary pressure which gives a direct indication of pulmonary status via pulmonary systolic and diastolic pressures.

Furthermore, the implanted device that measures the left and right atrial pressures can be used in combination with other medical devices. Examples of such medical devices include, but are not limited to, blood pressure cuffs, pulse-oximeters, scales, creatinine testing devices, smart devices, and wearable medical tracking devices, to name a few. The measurement device 41 can also be combined with other implantable devices, such as a ventricular assist device (VAD), drug delivery shunt or system, or other device. The measurement device 41 may provide feedback to the other implantable device(s), as part of a closed loop or open loop feedback system. The VAD may be a right VAD, a left VAD, or a bi VAD.

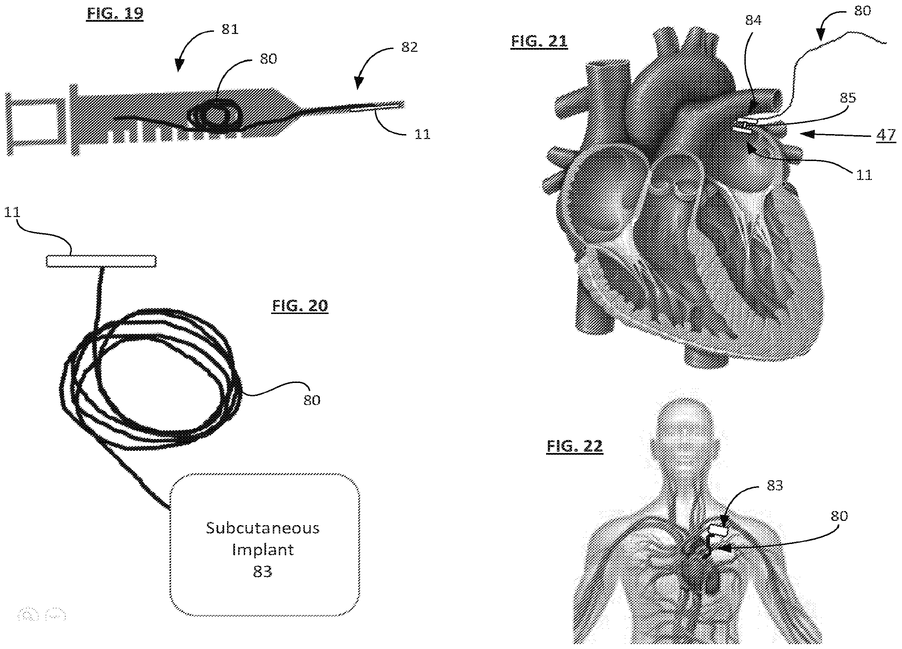

FIGs. 19 to 22 show additional sensor element examples as well as the associated delivery systems and methods. As shown, the left side sensing element 11 may be coupled with a tether 80, with the sensor element 11 and tether 80 configured to delivered through a needle delivery system 81 shown schematically in FIG. 19. Generally, the delivery system 81 may include a catheter and associated delivery needle 82 at a distal end of the catheter that is used to access a target area in the body (e.g., via ultrasound, radiographic, optical or other guidance). For example, the needle may be used to puncture a wall of the heart (e.g., left atrial wall) and the left side sensing element 11 may be advanced through the needle 82 into the target space (e.g., the left atrium). The needle 82 may then be retracted and the sensing element 11 may be pulled or tensioned against an inner wall of the heart (e.g., the inner wall defining the left atrium). A pledget or other anchor 84 may be advanced (e.g., over the tether 80) to a position on an opposite side of the heart wall from the sensing element 11 (e.g., a location on an outer wall of the heart proximate to the location of the sensing element 11) to help secure the sensing element 11 in place (e.g., as shown in FIG. 21). The tether 80 may be connected to a subcutaneous implant 83 that handles power, signal processing, and data transmission functions as shown in FIG. 20.

The subcutaneous implant 83 can include a battery, an antenna, and a control module (e.g., a microchip) to help control data collection and communication functions. In one example, the measurement device 47 may include a plug 85 that is placed between the sensing element 11 and the pledget 84 to help fill the fenestration left by the needle 82.

FIG. 22 shows a subcutaneous implant 83 on the other end of the tether 80 opposite from the sensing element 11. Multiple sensor elements similar to the left side sensing element 11 can be placed in any different parts of the heart as deemed suitable by the medical service providers. For example, after the left side sensing element 11 is placed in the left atrium, another sensing element such as the right-side sensing element 10 can be implemented into the right atrium using the same technique that is used to place the sensing element 11 in the left atrium. Another similar sensing element can be implanted in the left ventricle, right ventricle, or any other location in the heart or vasculature as desired. As such, any number of sensor elements can be implemented by penetrating a wall of the heart to take measurements (pressure, temperature and/or oxygen saturation) in any of the chambers of the heart or associated vasculature.

The tether(s) associated with the sensor elements can be coupled to the same subcutaneous implant or different subcutaneous implants as desired. Whether a single subcutaneous implant with data receiving and communication capabilities or different subcutaneous implants, it should be understood that any of the combination of measurements (pressure, temperature and/or oxygen saturation) at any combination of locations (e.g., left atrium, right atrium, left ventricle, and/or right ventricle) may be realized using the tethered sensor elements described in association with FIGs. 19 to 22.

The pressure measurement data obtained using the sensing elements 10,11,13,15 as described herein can be used to perform pulse-contour method, which is another method that is used to measure the cardiac output of the patient. This method uses the continuous pressure measurement data to plot a pressure-versus-time graph for the patient's heart, after which the pressure integral, i.e. the area beneath the plotted line on the pressure-versus-time graph, is used to determine the stroke volume (SV) of the portion of the heart that is being measured. The value of SV multiplied by the heart rate is the cardiac output.

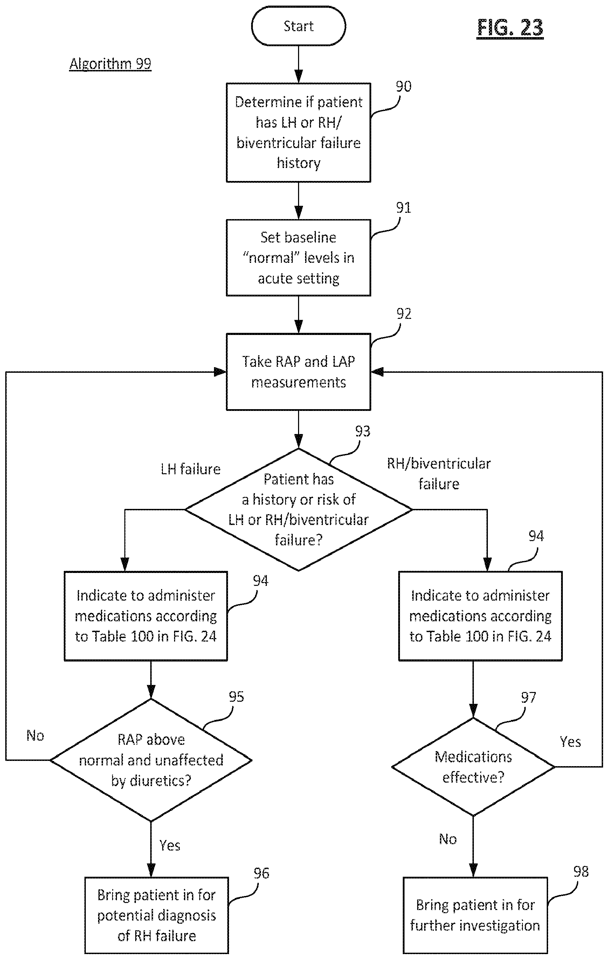

FIG. 23 is a flow chart showing a remote medical treatment monitoring method 99 that can be implemented using one or more electronic devices, such as the monitoring system 72, using measurement data received from the measurement device 41, for example, or any of the sensor elements described herein. In some examples, the method 99 is used for patients with a history of left heart failure (LHF), to determine treatment protocols guided by measured right and left heart physiologic metrics (e.g., pressure, temperature, and/or oxygen saturation).

Regardless, in some embodiments, in an optional first step 90 the service provider determines if the patient receiving treatment has a history of either left heart (LH) or right heart (RH)/biventricular failure. The method 99 may be used for patients with a risk of LH or RH/biventricular failure as determined by the medical service providers, regardless of history. In optional step 91, the medical service provider set a baseline "normal" level for applicable physiologic metrices (e.g., the left and right atrial pressures) in the acute setting by performing various tests on the patient to determine, based on the current condition of the patient, what normal levels (pressure, cardiac output, and/or oxygen saturation) would be. Baseline values can then be entered into the system which transfers the data to the monitoring system 72. In the example illustrated in this figure, the pressures being measured are the left atrial pressure (LAP) and the right atrial pressure (RAP). Other embodiments may include other measurements of other parts of the heart, as deemed appropriate by the medical service provider.

In some examples, the monitoring system 72 receives RAP and LAP measurements from the sensors in step 92, such as the right-side sensing element 10 and the left side sensing element 11. In one implementation, the measurements include whether the pressure values of the right atrium and the left atrium are trending below, at, or above the normal level. In another example, the method may also consider whether the pressure values are increasing, decreasing, or staying steady as an additional input into the overall assessment.

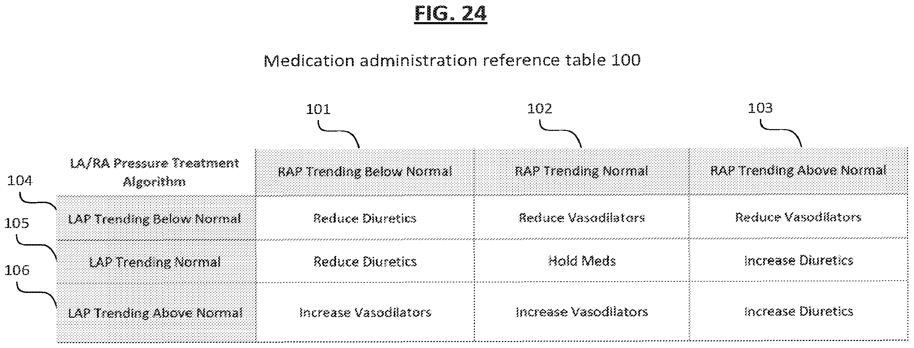

In optional step 93, the monitoring system 72 confirms whether the patient has a history of LH or RH/biventricular failure. The monitoring system 72 optionally uses a medication administration reference table 100 in FIG. 24 to determine and indicate if dosage of certain medications needs to be increased or reduced, in step 94. Alternatively, a medical service provider (e.g., physician) optionally uses the data directly to assess what treatment regimen (e.g., pharmacological) is appropriate based upon the data using the methodology of table 100.

As shown, the table 100 has three columns and three rows, where the columns pertain to "RAP trending below normal" 101, "RAP trending normal" 102, and "RAP trending above normal" 103, and the columns pertain to "LAP trending below normal" 104, "LAP trending normal" 105, and "LAP trending above normal" 106. For example, if the RAP is trending below normal but the LAP is trending above normal, the method would include the step of "Increase Vasodilators" according to the table 100. If automated, a consistent "message" or communication could be relayed to a user of the monitoring system. On the other hand, if the RAP is also trending above normal, the method would include the step of "Increase Diuretics". Again, if automated, a consistent "message" or communication could be relayed to a user of the monitoring system. It should be noted that when the LAP and RAP values are both in the normal level (i.e. the box defined by the "LAP normal" row and "RAP normal" column), one method would include not altering any medications.

After the initial medication is administered, the method 99 includes verifying to see if the RAP is still trending above normal and if the RAP value is unaffected by diuretics, in step 95. This may occur in the second example shown above, where the LAP and RAP are both trending above normal, so the amount of diuretics administered to the patient is increased, but a subsequent measurement of the RAP shows that this pressure is still above normal. In this instance, the monitoring system 72 could display an indication in step 96 instructing the medical service provider to bring the patient in for a potential diagnosis of RH failure (or the medical service provider could carry out the step 96 based upon the data). Among other possible causes of high RAP is primary pulmonary arterial hypertension. When the medical service provider tests the patient for possible diagnosis of these conditions, the medical service provider can set a new baseline value range for the "RAP normal" level and update the patient's status as having a history of RH/biventricular failure so that moving forward, the method will proceed to step 97 instead of step 95 in the future. Otherwise, if the RAP decreases to the normal level, the monitoring system 72 optionally goes back to step 92 to take subsequent RAP and LAP measurements.

Returning to step 93, if the monitoring system 72 (or the medical service provider) confirms that the patient has a history of RH/biventricular failure, the method 99 proceeds to step 97 after determining which medication to increase or decrease based on analysis outlined in table 100. In step 97, the method 99 includes determining if the medication administered in step 94 is effective. For example, the method 99 may include comparing the previous LAP and RAP values with the new LAP and RAP values taken after the medication is administered. If the comparison shows that there is an insufficient change in the status in a way that indicates that the administered medication is ineffective (for example, if the LAP or RAP is still below normal and the medication is not causing it to increase toward normal level, or if the LAP or RAP is still above normal and the medication is not causing it to decrease toward normal level, etc.) the medical service provider may bring the patient in for adjusted treatment and/or the monitoring system 72 may provide a message or other communication indicating that further diagnosis / treatment is warranted in step 98. The possible lack of efficacy of the medications may be a sign of increased exigency or that immediate medical attention is otherwise warranted. Otherwise, if the administered medication is showing apparent efficacy in moving LAP and RAP toward nominal or desired levels, the method returns to step 92 and the monitoring system 72 continues to receive and evaluate new measurements for assessing patient health.

Use of at least two sets of measurement data (in this example, LAP and RAP measurements) in assessing cardiac function is advantageous over prior-art methods with only one set of measurement data for a variety of reasons, including that the second set helps facilitate more accurate root cause diagnosis and treatment.

In another embodiment, the method 99 may be programmed so that instead of using the actual measured LAP and RAP values, a ratio of LAP to RAP (or a ratio of RAP to LAP) may be used to determine which medications to administer and how much. This methodology may be based on the understanding that the pressures within the left and right atria should correspond to a desired ratio (e.g., 2:1 LAP: RAP) in a healthy heart, therefore the ideal ratio of LAP to RAP can be determined (e.g., an ideal ratio of 2:1 pressures are desired), and any ratio that is significantly smaller or larger than the desired ratio (e.g., 2:1) would pose a threat to the patient's health.

In some examples, if the ratio of LAP to RAP is above a threshold value (i.e. the LAP is much higher than the RAP) and keeps increasing in a patient with a history of LH failure, the method may include a determination that the amount of vasodilators being administered should be increased. The threshold ratio value of LAP to RAP which triggers such a determination may be determined and updated periodically by the medical service provider (e.g., after examination performed on the patient). In other words, various methods include one or more medical service providers determining the range of "normal" baseline ratios, which will then be used in the medication administration reference table. Alternatively, a generalized set of guidelines may be provided to medical service providers regarding an appropriate baseline.

The method 99 can be adjusted to be more specific in terms of how much a pharmacological, or medication regimen needs to be increased or reduced, which can be varied based on how much the LAP and RAP are trending above or below the normal level. This may be done by implementing another table or set of guidelines within the table 100 that indicates the amount of medication to be administered (e.g., so that a treatment dosage may be adjusted for a patient without requiring direct medical service provider intervention). The table 100 can include any of a variety of medical recommendations / indications, such as beta-blockers and inotropes, for example, as indicated by a particular set of physiologic measurements and associated guidance of the table 100. Furthermore, to inform the patient on which medication to choose and its dosage, the type of medication (e.g. diuretic or vasodilator) that needs to be administered and the dosage thereof can be displayed on, for example, the screen of a computer or a display of a smart device used by the patient.

As referenced above, the measurement data and associated monitoring and treatment methodology is not necessarily limited to LAP and RAP measurements. In some examples, additional or alternative locations (e.g., pulmonary arteries, ventricles, pulmonary veins, aorta, and others) and/or additional or alternative metrics (e.g., temperature and/or oxygen saturation) may be utilized in implementing a monitoring and treatment method such as the method 99.

As explained above, the method 99 may be performed manually or may be partially or completely automated using any device capable of receiving and processing the measurement data from the measurement device 41. For example, the method 99 may be implemented entirely in the monitoring system 72 (e.g., such as a smart device), which performs all the comparisons, calculations, and determinations after receiving the LAP and RAP measurement data from the measurement system 41. In some examples, the method may be implemented partially in the monitoring system 72 and partially in the communications relay 70 which may include a processing unit to receive the LAP and RAP measurement data from the sensors, determine whether the LAP and RAP are above/at/below normal level and decreasing/steady/increasing, then relay this information to the remote device 72 to perform the rest of the method. In yet another example, the subcutaneous implant 83 may be programmed to perform a portion or the entirety of the method.

In still further examples, the method 99 may be implemented in a device with a user interface allowing the patient to administer medications according to the results of the method. The method may also be implemented in the medical service providers' electronic health record (EHR) or electronic medical record (EMR) systems which keep track of the necessary records of each patient. As such, the EHR or EMR systems may use local or remote database to access, among other things, the patient's history of LH or RH/biventricular failure and whether the medical service providers have deemed the patient to be at a risk of such failure. The resulting data from the method may be displayed on a dashboard of the user interface with multiple options for the user (e.g. patient and medical service providers), which may include: LAP and RAP averages, trend arrows, line graphs over time, and waveforms, as well as a history of the medications taken by the patient, etc. The dashboard may also be configured such that the user can first pull up the most meaningful information, such as the averages and trends, then dig in further for a more detailed analysis, such as the waveforms. This may be implemented by organizing the multiple options in a hierarchical manner based on the importance of each option. In one example, this hierarchical order of the options is customizable according to the user's preference, such that the most preferred information can be pulled up first.

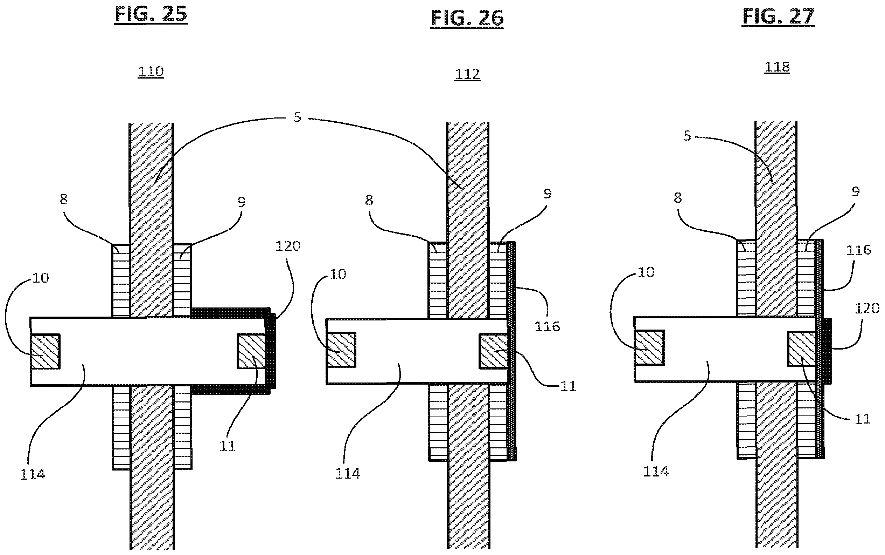

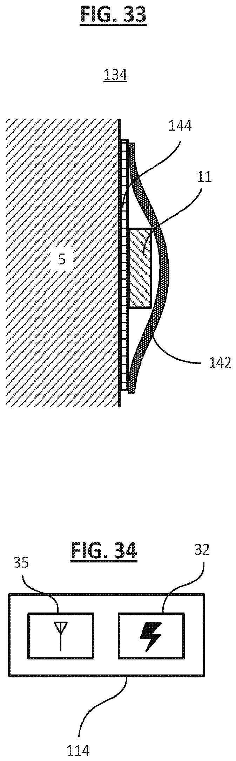

FIG. 25 shows an embodiment of a measurement device 110 according to the present disclosure. The measurement device 110 has an electronics housing component 114 which stores an antenna, for example the antenna coil 35, and the battery or power source 32, as shown in FIG. 34, such that after the measurement device 110 including the housing component 114 is inserted through an aperture formed on the atrial septum 5, the housing component 114 protrudes from and extends beyond both walls of the septum 5 and the anchoring discs 8,9. The antenna 35 transmits the measurement data taken by the sensing elements 10,11 to an external monitoring system (not shown) which receives, tracks, and performs analysis of the data. The measurement device 110 has the right anchoring disc 8 and the left anchoring disc 9 working together to help hold the housing component 114 in place so that the housing component 114 is secured in position relative to the septum 5. As shown in the figure, the two discs 8,9 are designed to sandwich the atrial septum 5 by either actively engaging or contacting each side in an opposing manner. In one example, the housing component 114 is made of a suitable metal such as titanium, stainless steel, or other biocompatible metal. In another example, the housing component 114 is made of plastic or other suitable polymeric material. In still other examples, the housing component 114 is formed of a biocompatible ceramic material, such as glass.

The sensing elements 10,11 are implemented or disposed in the housing component 114 such that these sensing elements 10,11 are located on the two ends of the housing component 114 that extended into the atria. In some examples, the sensing elements 10, 11 protrude into the atria to enable better sensing functionality. For example, in some examples, it may be more beneficial to have the sensing elements 10, 11 located at the two ends of the housing component 114 to space the sensing elements 10, 11 from the septum 5 in order to prevent tissue ingrowth from affecting the measuring efficacy of the sensing elements 10, 11.

One advantage of having the sensing elements 10,11 protruding into the atrium and raised above the surface of the septum 5 is that the sensing elements 10,11 may perform more effective (e.g., accurate) physiologic measurements chronically than if the sensing elements 10,11 were located relatively flush with the septum 5. In some examples, a layer of anticoagulant agent 120, as shown in FIG. 25, is disposed over a portion or an entirety of a surface proximate one or both of the sensing elements 10, 11 to effectively reduce the risk for thrombus forming on the device or due to turbulence of flow that could lead to stroke or embolic events. The layer of anticoagulant agent 120 may be positioned over one or more of the housing component 114, the sensing elements 10,11, and/or the anchoring discs 8,9, for example.

Possible anticoagulant agents which may be used include but are not limited to: heparin, warfarin, rivaroxaban, dabigatran, apixaban, edoxaban, enoxaparin, and fondaparinux. In one example, the layer 120 may be a layer of antiinflammatory agent such as dexamethasone to reduce inflammation of the tissues proximate to the sensing elements 10,11 to improve accuracy of the measurements. In some examples, the layer of anticoagulant agent 120 is formed by a CBAS^{®} Heparin Surface treatment available from W.L. Gore & Associates, Inc.

FIG. 26 shows an embodiment of a measurement device 112 according to the present disclosure. In the measurement device 112, the housing component 114 is positioned such that one end of the housing component 114 is substantially flush with the corresponding anchoring component, in this example the anchoring disc 9, thereby reducing the amount of flow disruption and subsequent potential for device related thrombus. As a result, there is a need to control the tissue growth to enable reliable, chronic sensor performance. As such, a layer of biocompatible material 116 is disposed on a portion or an entirety of a surface of one or more of the housing component 114, the sensing elements 10,11, and/or the anchoring discs 8,9. The biocompatible material with a suitable structure which controls tissue ingrowth, such as expanded polytetrafluoroethylene (ePTFE), for example. Other examples of the biocompatible material include but are not limited to suitable polymeric or synthetic materials or naturally occurring materials such as polyethylene terephthalate (PET), polyethylene (PE), polypropylene (PP), Silicone, polylactic acid (PLA), polyglycolide (PGA), Polyglycolic Acid:Trimethylene Carbonate (PGA:TMC), Stainless Steel, Nitinol, decellularized tissue matrix, fluorinated ethylene propylene (FEP), copolymers of tetrafluoroethylene (TFE) and perfluoro(propyl vinyl ether) (PFA), homopolymers of polychlorotrifluoroethylene (PCTFE), and its copolymers with TFE, ethylenechlorotrifluoroethylene (ECTFE), copolymers of ethylene-tetrafluoroethylene (ETFE), polyvinylidene fluoride (PVDF), and polyvinyfluoride (PVF). Other examples of the structures include but are not limited to suitable wovens, non-wovens, braids, knits, films, or microporous films including electrospun webs, expanded polymers, foams, melt-blown webs, etc. The tissue whose ingrowth is promoted may be vascular endothelial cells.

FIG. 27 shows an embodiment of a measurement device 118 according to various examples. The measurement device 118 further includes the additional layer of anticoagulant agent 120 on top of the layer of biocompatible material 116 from FIG. 26. In one example, the layer 120 is of sufficient size to cover the surface of the sensing element 11 such that the anticoagulant agent hinders tissue ingrowth over the sensing element 11, thereby minimizing errors in the physiologic measurements performed by the sensing element 11 or otherwise enhancing efficacy of the sensing element 11 over time (e.g., precision and / or accuracy).

In another example, the layer 120 can cover a smaller or larger area of the layer 116 depending on the implementation. Other examples of film layer can allow selective tissue ingrowth such that the tissue ingrowth is permitted or encouraged around a perimeter portion of the anchoring disc 9 (or portions thereof) while providing a "window" or portion with lesser or no tissue ingrowth corresponding to the area on and/or surrounding the sensing element 11. For example, the layer of biocompatible material 116 can have the anticoagulant agent imbued or incorporated into a certain area so the tissue ingrowth is reduced or minimized in that certain area to optimize function of the sensing element 11. In view of the above, different combinations of agents and polymers may be used to adjust the amount of tissue ingrowth that is achieved such that there is sufficient tissue ingrowth and anchoring but not so much that the tissue hinders effective measurement by the sensing elements 10,11.

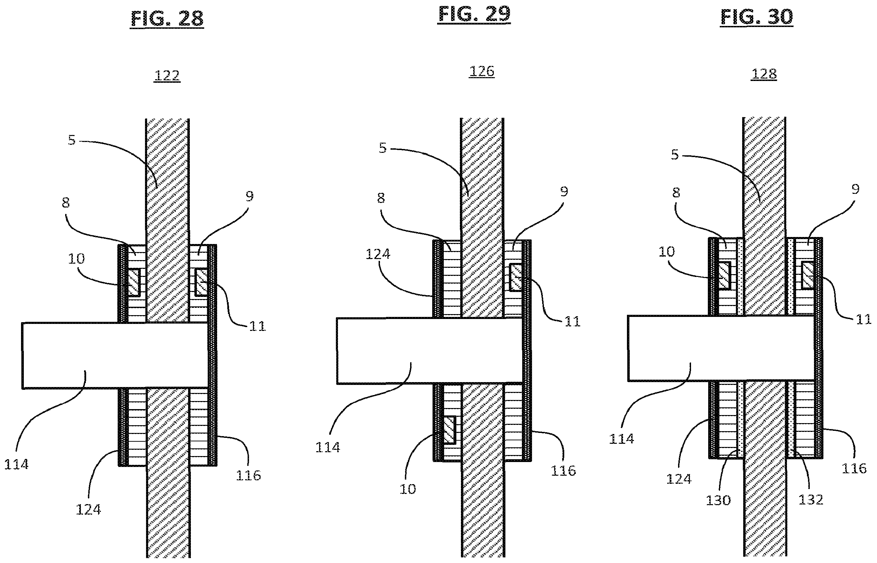

FIG. 28 shows an embodiment of a measurement device 122 according to the present disclosure. The measurement device 122 the sensing elements 10,11 are disposed in the anchoring discs 8,9, respectively. The surface of the disc 9 and the sensing element 11 is covered with the layer 116 as previously explained, and the surface of the disc 8 and the sensing element 10 is covered with another layer 124 of the same biocompatible material as the layer 116 so that tissue ingrowth is promoted over both discs. In one example, the biocompatible material used may differ between the two layers 116, 124. In some examples, the housing component 114 extends through the second layer 124 but is substantially flush with the first layer 116.

The sensing elements 10,11 in some examples are located or otherwise positioned relative to one another such that the sensing elements 10,11 align with each other across, or on opposite sides of, the atrial septum 5. That is, the distance between the sensing elements 10,11 is reduced to help minimize the overall surface area of the septum taken up by the sensing elements 10,11. One advantage of this configuration is that the remaining space occupied by the discs 8,9 that are not occupied by the sensing elements 10,11 is available in the event that an opening or aperture (fenestration) is to be formed on the atrial septum 5 to facilitate access across the septum (e.g., as part of a device delivery from the right to left side of the atrium), selective fluid flow, or for other purpose as desired.

As previously shown in FIGs. 14-16, the anchoring discs 8,9 may have re-crossable surfaces such that the opening or aperture can be formed without interfering with the electronics. In one example, the measurement device 112 can act as an intra-atrial shunt if placed between the left and right atria of the heart. FIG. 29 shows an embodiment of a measurement device 126 where the sensing elements 10,11 are located such that the positions of the sensing elements 10,11 are offset with each other across the atrial septum 5, and therefore are generally not aligned across the septum. In one example, an angle measured between the positions of the sensing elements 10,11 with respect to a longitudinal axis of the housing component 114 (or angular offset) may be less than 15 degrees, between 15 and 30 degrees, between 30 and 45 degrees, between 45 and 60 degrees, between 60 and 90 degrees, between 90 and 135 degrees, and between 135 and 180 degrees, for example, although a variety of arrangements are contemplated. FIG. 29 illustrates an example in which the angle is 180 degrees, and this configuration achieves maximum distance (or angular offset) between the sensing elements 10,11.

FIG. 30 shows an embodiment of a measurement device 128 according to the present disclosure, which includes two additional layers 130,132 of the biocompatible material. The layer 130 is disposed between the disc 8 and the septum 5, and the layer 132 is disposed between the anchoring disc 9 and the septum 5. In one example, the biocompatible material used in the additional layers 130,132 is the same as the biocompatible material used in the previously mentioned layers 116,124, though this need not be the case in other examples. In one example, the sensing elements 10,11 in any of the embodiments of FIGs. 28-30 may be attached, tethered, stitched, bound, stapled, or adhered to the anchoring discs 8,9. In another example, the anchoring discs 8,9 are formed with the sensing elements 10,11 as an integral component within the discs 8,9. Furthermore, in one example, the sensing elements 10,11 are coupled wirelessly (including but not limited to Bluetooth technology) with the electronics within the housing component 114 (e.g., the antenna 35 in FIG. 34). In another example, the sensing elements 10,11 are coupled via one or more tethers with the electronics.

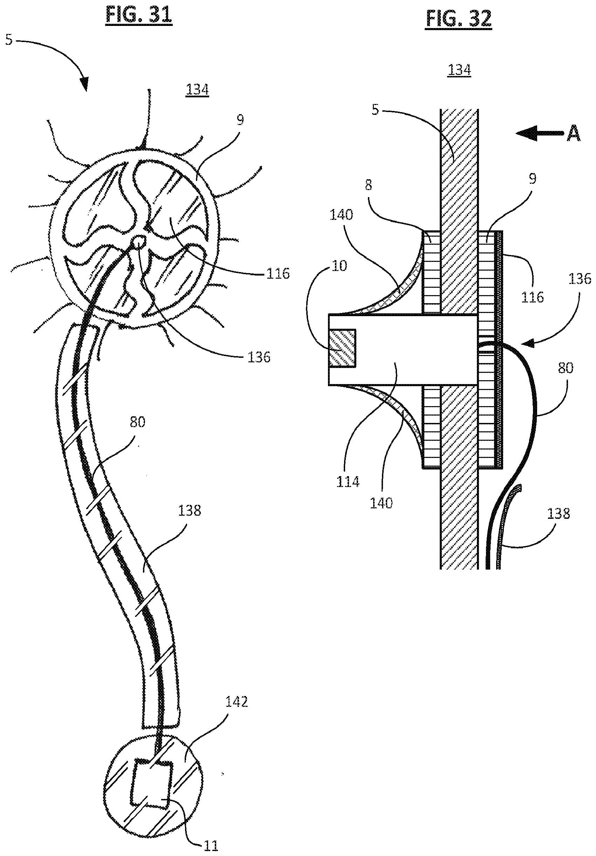

FIGs. 31 and 32 show another embodiment of a measurement device 134 according to some examples. FIG. 32 is a side view, whereas FIG. 31 shows the view from inside one of the atria adjacent to the atrial septum 5 as seen from the direction of the arrow A in FIG. 32. The measurement device 134 has the anchoring disc 9 placed and attached against the wall of the septum 5, and the disc 9 has an opening 136 through which the tether 80 extends from the electronics housing component 114 located behind the disc 9 into the atrium and ending at the sensing element 11. In this example, instead of being located inside the anchoring disc 9 or in the housing component 114, the sensing element 11 is decoupled from the housing component 114 and located at a location remote from the disc 9 and the housing component 114. In another example, the measurement device 134 can have a sensing element in the disc and another sensing element in a remote location, such that more physiologic measurements can be performed using the additional sensing element. In some examples, the tether 80 passes from an atrium to a ventricle, or a distal artery/vein, such that the sensing element 11 performs physiologic measurements inside the ventricle.

The tether 80 can be covered partially or entirely by a layer 138 of biocompatible material. The layer 138 may be configured to promote tissue ingrowth and may be of sufficient length to help prevent the tether 80 from detaching from the surface of the septum 5. In another example, instead of a single elongated layer 138, there may be multiple separate layers covering the tether 80 at different sections thereof. Also, the sensing element 11 may be covered by a layer 142 of biocompatible material. In some examples, the layer 142 is optionally configured similarly to the layer 116 referenced herein. In one example, similar to the embodiment shown in FIG. 30, the layer 132 of biocompatible material may be disposed between the sensing element 11 and the septum 5.

FIG. 33 shows an embodiment in which the sensing element 11 is sandwiched between two layers of biocompatible material, one of which may be the layer 142 previously mentioned. In the shown embodiment, the sensing element 11 is sandwiched between a bottom layer 144 and a top layer, which in this case is the layer 142. The proximal layer 144 may be positioned on the opposite side of the sensing element 11 from the layer 142 such that the sensing element 11 does not directly come into contact with a surface of the septum 5. In one example, the two layers 142, 144 sandwiching the sensing element 11 cover the same surface area such that the top layer 142 completely covers the other layer 144 to laminate the sensing element 11. In another example, the two layers 142, 144 have different surface areas such that when they are overlapped with one another, one of the layers extends beyond the other layer. In one example, the two layers 142, 144 are made of the same biocompatible material, whereas in another example, these layers may be made of different biocompatible materials.

In some examples, the right anchoring disc 8 is disposed in the right atrium and the left anchoring disc 9 is disposed in the left atrium of the heart, such that electronics housing component 114 of the measurement devices 112, 118, 122, 126, and 128 all extend into the right atrium of the heart while being substantially flush with the left anchoring disc 9 in the left atrium. In other examples, the housing component 114 may extend into the left atrium of the heart instead, such that the housing component 114 is substantially flush with the right anchoring disc 8 in the right atrium. In some examples, because of the imbalance created between the protrusion on one end and the flush surface on the other end of the housing component 114, one or more reinforcement struts 140 are employed to better support the housing component 114 with one or both of the anchoring discs 8,9 to help stabilize the implant to prevent unwanted device motion leading to increased inflammatory response and tissue growth and to prevent the housing component 114 from unwanted flexing and/or decoupling. FIG. 32 shows the reinforcement struts 140 supporting the housing component 114 against the anchoring disc 8. In some examples, the reinforcement struts are nitinol wires or any other suitable material capable of stabilizing the housing component 114.

In one example, one or more of the layers of biocompatible material employed is hydrophobic. In such examples, one or more of those layers may be covered, coated, imbibed, or otherwise associated with a hydrophilic material such that the material is relatively more "echolucent". For reference, a material that is echolucent allows passage of ultrasonic waves therethrough such that the material does not interfere with a sonogram, or ultrasound. A microporous membrane, such as ePTFE, can also be modified to be echolucent by incorporating a vinyl monomer, such as PVA, polymerized within the pores of the membrane. The hydrophilic material helps reduce or eliminate the presence of air in the material, which in turn decreases the interference between the air and the ultrasound wave transmission. Other examples of hydrophilic layers formed by applying a polymeric hydrophilic surfactant are taught in U.S. Patent No. 7,871,659 to Cook et al., assigned to Gore W. L. and Associates Inc.

In some examples, the properties of one or more components of the measuring device, including the anchoring discs, layers of biocompatible material, and/or sensing elements, can be adjusted to better suit the physiologic measurements being performed. For example, the sensing element may be a flow sensor (e.g., ultrasound or other type of flow sensor), a temperature sensor, a pressure sensor, combinations thereof, or other sensor types as desired. Thus, the materials employed for the layers of biocompatible material may be configured to promote or otherwise effectively transfer the mechanical / hydraulic response, thermal response, or other response to physiologic conditions in the portion of the heart, or other area of the body, being measured.

The sensing element may be highly thermally conductive, have a high level of hydraulic conductivity, or otherwise facilitate sensitivity to changes in the environment being measured by the sensor. In addition to the examples describing pressure measurements, the various measurement devices may be configured to take temperature measurements to monitor cardiac function, for example. In some methods of monitoring, a cold bolus (e.g., fluid) may be introduced into the cardiac system and the rate of temperature equalization may be used to determine cardiac output within the heart, which is then measured by the thermometer. In such instances, the components may need to have thermally conductive properties to convey heat from the environment for sensing the changes in temperature.

The sensing element may be an oxygen saturation sensor which measures the diffusion characteristics of oxygen within the atrium. In such instances, the components may need to be sufficiently exposed to the environment to be able to detect such changes in the diffusion characteristics or be configured to effectively pass oxygen to the sensing components. These are just a few examples, and additional examples include adjusting other physical properties of the various layers and components of the measurement devices to improve sensor function.

Furthermore, in some examples, the sensing element or portions of the measurement devices may incorporate flexible, printed electronics including patterned traces deposited on one or more layers (e.g., to provide an antenna, inductive power source, communication or signal traces, or other functions. In one example, such patterned traces connect the sensing element to the housing component in lieu of the tether as part of the sensor assembly. In another example, such patterned traces are configured to provide sensing data, or otherwise take measurements.

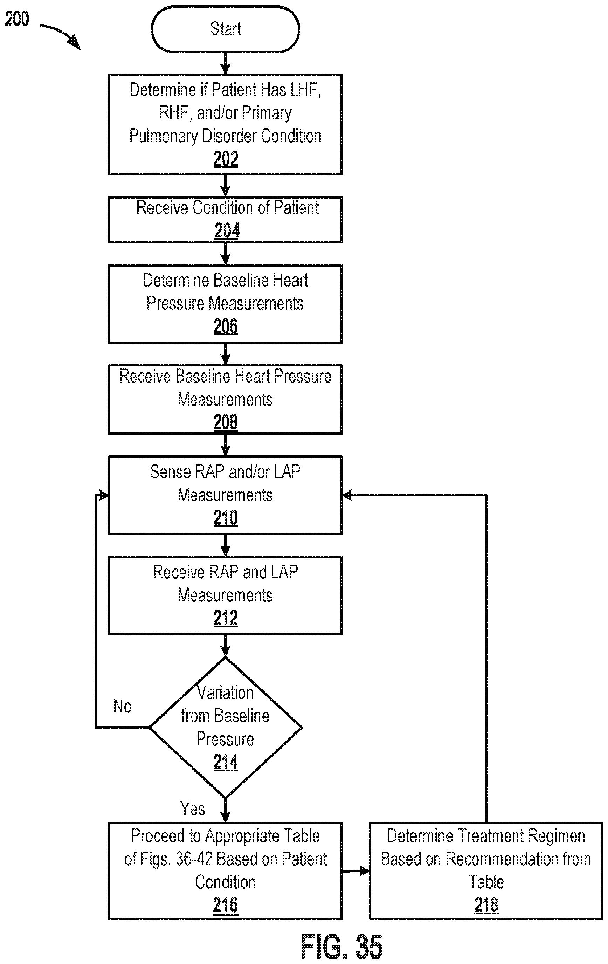

FIG. 35 illustrates a flow diagram of a method 200 for determining a treatment regimen for a patient according to some embodiments. In at least some embodiments, the treatment regimen for the patient may include maintaining a dosing regimen for the patient, increasing the dosing regimen for the patient, decreasing the dosing regimen for the patient, changing the type of medication for the patient, confirming and/or checking the heart waveform for the patient, and/or suggesting the patient visit a medical professional for further testing and/or diagnosis. In at least some embodiments, the treatment regimen for the patient may be communicated to the patient by the monitoring system 72. In some embodiments, the embodiments described below for changing the type of medication for the patient may be determined by a processing device using machine learning techniques.

For example, the method 200 may be implemented using one or more electronic devices, such as the monitoring system 72, using measurement data received from, for example, the measurement device 41 or any of the sensor elements described herein. In at least some embodiments, the method 200 may be used for patients with a history of LHF, right heart failure (RHF), and/or primary pulmonary disorder to determine a treatment regimen guided by sensed left heart pressure measurements (e.g., left atrial pressure measurements) and/or right heart pressure measurements (e.g., right atrial pressure measurements).

In at least some embodiments, the method 200 (and/or algorithm 99) may be used in a closed loop system (e.g., diuretic and/or vasodilator pump) to reduce the need to rely on patient compliance. Additionally, or alternatively, the method 200 (and/or algorithm 99) may be used with and/or incorporated into a therapy device (e.g., VAD) to adjust device settings (e.g., VAD RPMs) in addition to medications.

In some embodiments, the method 200 includes determining if the patient has LHF, RHF, and/or primary pulmonary disorder condition (block 202). In at least some embodiments, a medical service professional may make the determination based on one or more of patient history, family history, a physical examination, chest radiography, electrocardiography, and/or the like. In embodiments, the method 200 may include inputting and/or communicating the condition of the patient to one or more devices (block 204). For example, the condition may be input into the monitoring system 72.

The method 200 may also include determining one or more baseline heart pressure measurements (block 206). In at least some embodiments, the baseline heart pressure measurements may be baseline left heart pressure measurements and/or baseline right heart pressure measurements. For example, the left heart pressure measurements may be left atrial pressure measurements (LAP) and the right heart pressure measurements may be right atrial pressure measurements (RAP). Additionally, or alternatively, other measurements may be sensed to represent the left heart and/or right heart pressures. For example, surrogates for the left heart pressure may be used. Exemplary surrogates for the left heart pressure may include, but are not limited to, the following: pulmonary artery pressure (PAP), pulmonary arterial wedge pressure (PAWP), pulmonary artery systolic pressure (PASP), pulmonary artery diastolic pressure (PADP), right ventricular systolic pressure (RVSP), estimated pulmonary artery diastolic pressure (ePAD), and left ventricular end-diastolic pressure (LVEDP). As another example, surrogates for the right heart pressure may be used. Exemplary surrogates for the right heart pressure may include, but are not limited to, the following right ventricular end-diastolic pressure (RVEDP), central venous pressure (CVP), and jugular venous pulse (JVP).

In at least some embodiments, a Valsalva pressure measurement technique (remote or in office) may be used to re-calibrate the pressure sensors if the pressure reading is suspect to sensor drift. The Valsalva technique allows the RAP and LAP to equalize with the exhale pressure of a patient within seconds. If RAP and/or LAP are offset from the exhaled pressured, then a sensor baseline reading can be re-established in the software. Due to the sensor location (atria), this technique will prevent the need for a right heart catheterization in order to recalibrate the sensors upon suspect of sensor drift.

In at least some embodiments, the baseline heart pressure measurements may be determined based on healthy heart pressure measurements. For example, a healthy heart may have left heart pressure measurements that are approximately L₁ and right heart pressure measurements that are approximately R₁. In embodiments, the baseline heart pressure measurements may be set to L₁ and R₁ +/- an appropriate variation. In at least some embodiments, the variation may be +/-10%, 20%, etc. As such, the baseline heart pressure measurements may be set to L₁ and R₁ +/- 10%, 20%, etc.

Additionally, or alternatively, the baseline heart pressure measurements may be determined by a medical service provider based on the condition of the patient. For example, a medical service provider may assign baseline heart pressure measurements based on the condition of the patient (e.g., LHR, RHF, and/or primary pulmonary disorder) and/or may test the baseline heart pressure measurements of the patient in an acute setting by performing various tests on the patient to determine, based on the current condition of the patient, what normal heart pressure measurements would be. The baseline heart pressure measurements can then be input into and received by the monitoring system 72 (block 208).

The method 200 may also include sensing the LAP and/or the RAP after baseline pressures are established (block 210). In at least some embodiments, the LAP and/or the RAP are sensed at regular intervals. For example, the LAP and/or the RAP may be sensed every minute, every hour, every day, every week, every month, etc. In some embodiments, the right-side sensing element 10 and the left-side sensing element 11 may be used to sense the RAP and/or the LAP, respectively. Once sensed, the LAP and/or the RAP may be sent to and received by the monitoring system 72 (block 212).

The method 200 may further include determining whether the sensed LAP and/or the sensed RAP vary from the baseline heart pressure measurements (block 214). For example, the monitoring system 72 may compare the LAP and/or the RAP sensed in block 210 with the baseline measurements established in block 208. The monitoring system 72 can then determine whether the LAP and/or RAP are below, at, or above the baseline heart pressure measurements established in block 208. For example, if the LAP and/or the RAP are within a threshold of the baseline heart pressure measurements, then the method 200 may proceed back to block 210 and continue to monitor the LAP and/or the RAP. Alternatively, if the LAP and/or the RAP are above or below the baseline heart pressure measurements by a threshold, then the method 200 may proceed to block 216.

In at least some embodiments, the threshold may be a percentage difference of the baseline heart pressure measurements. In some embodiments, the percentage difference may be input into the monitoring system 72. For example, the threshold may be +/- 5%, +/- 10%, +/- 15%, +/- 20%, +/- 25%, etc. of the baseline heart pressure measurements. And, once a percentage threshold is selected and input into the monitoring system 72, if the sensed LAP and/or the sensed RAP are within the selected percentage of the baseline heart pressure measurements, then the method 200 may proceed to block 210. Alternatively, if the sensed LAP and/or the sensed RAP differ by the selected percentage or differ by more than the selected percentage from the baseline heart pressure measurements, then the method 200 may proceed to block 216.

In at least some other embodiments, the threshold may be a constant. In some embodiments, the percentage difference may be input into the monitoring system 72. For example, the threshold may be x₁ millimeters of mercury (mmHg). And, if the sensed LAP and/or the sensed RAP are within x₁ of the baseline heart pressure measurements, then the method 200 may proceed to block 210. Alternatively, if the sensed LAP and/or the sensed RAP differ by x₁ or differ by more than x₁ than the baseline heart pressure measurements, then the method 200 may proceed to block 216.

Additionally, or alternatively, the method 200 may also include determining a trend of the sensed LAP and/or the sensed RAP. For example, the monitoring system 72 may determine whether the pressure values are increasing, decreasing, or staying steady as an additional input into the overall assessment.

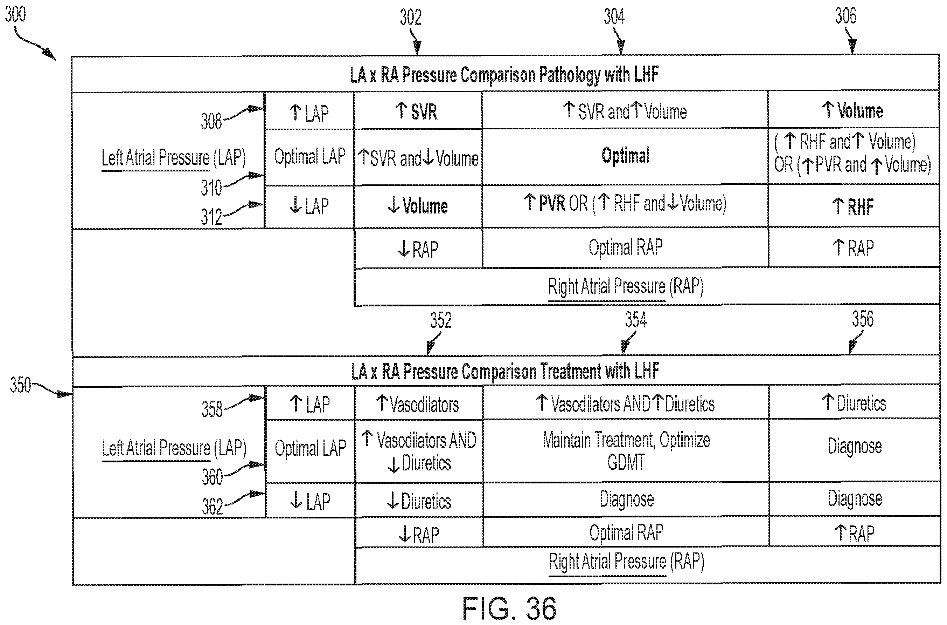

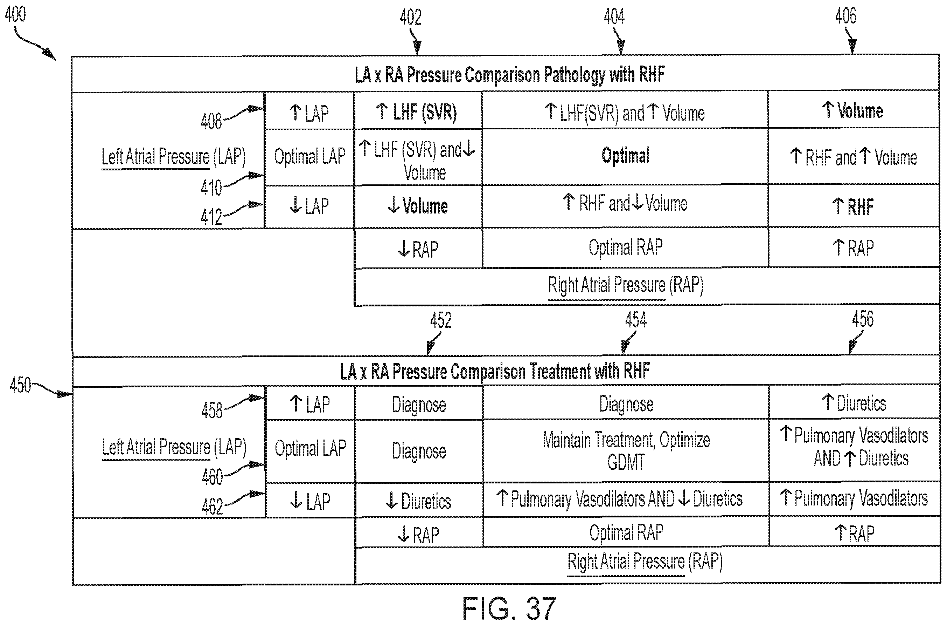

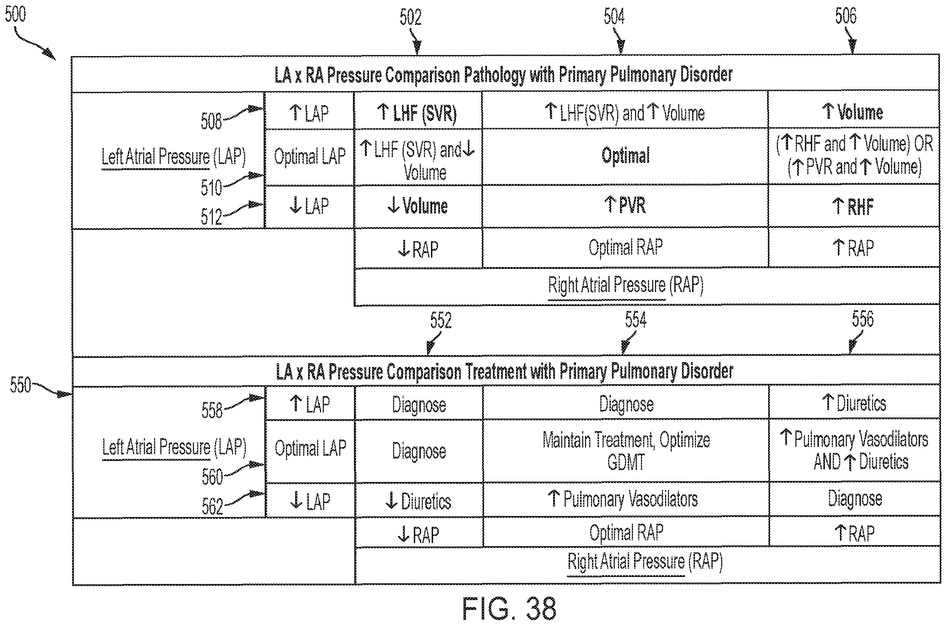

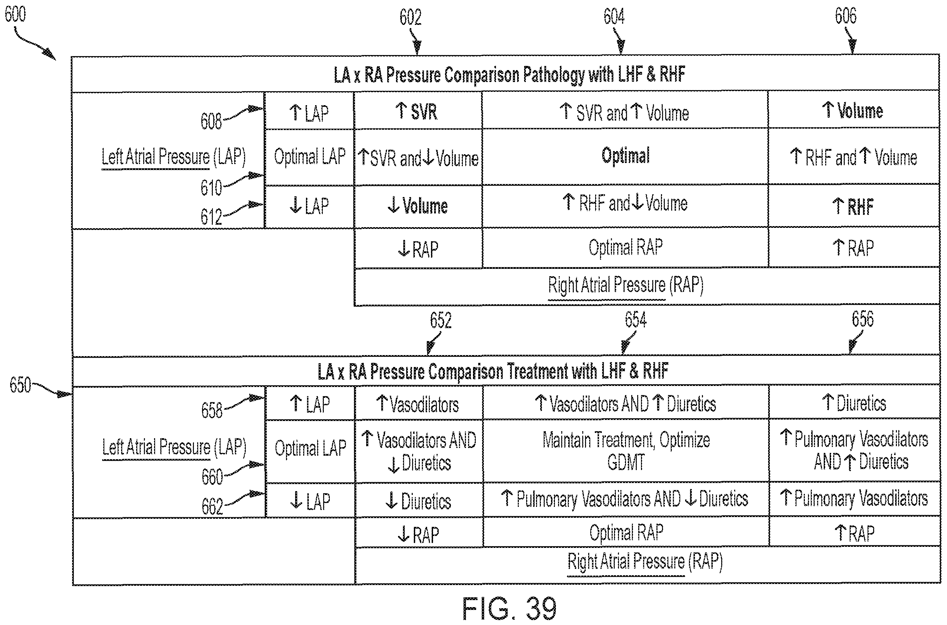

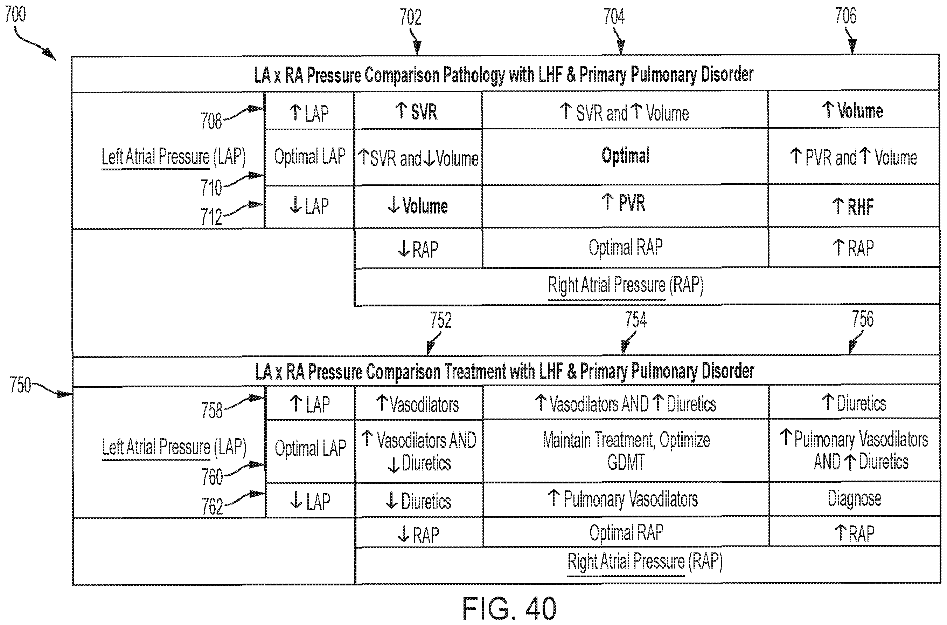

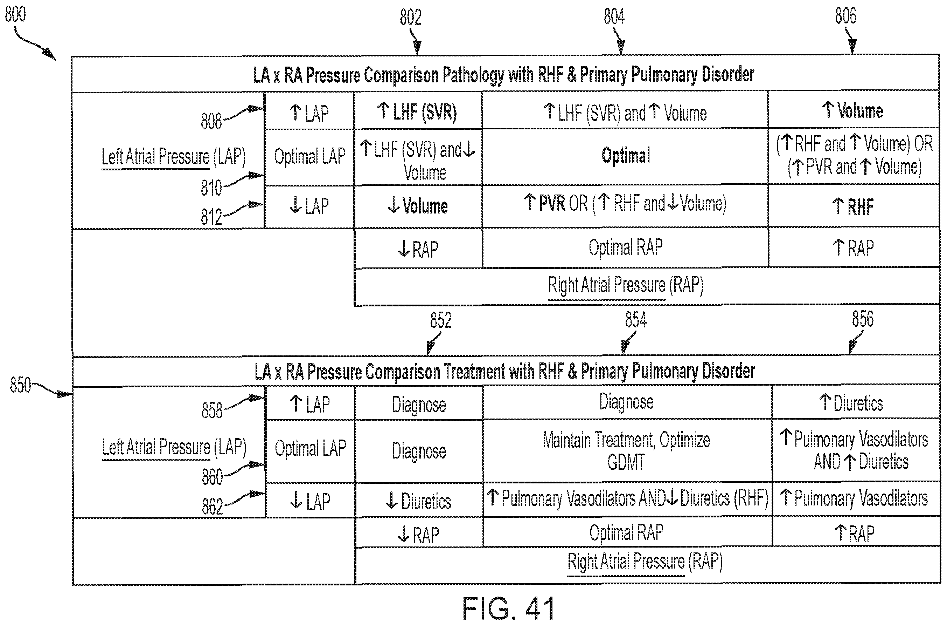

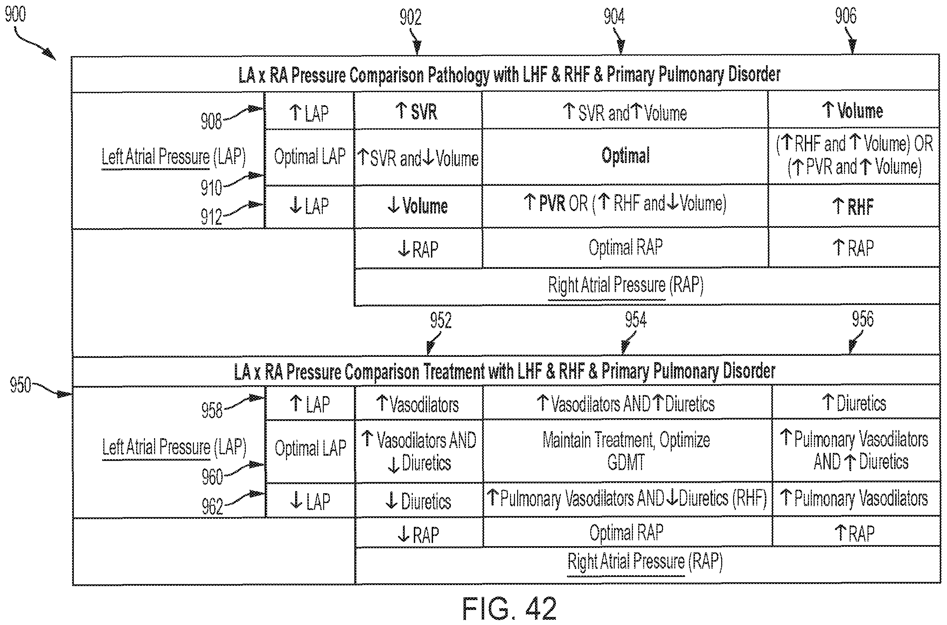

In the event the method 200 proceeds to block 216, the method 200 proceeds to appropriate figure of FIGs. 36-42, based on the patient's condition. That is, if the method 200 determines the patient has only LHF at block 202, then the method 200 proceeds to tables 300, 350 illustrated in FIG. 36. If the method 200 determines the patient has only RHF at block 202, then the method 200 proceeds to table 400, 450 illustrated in FIG. 37. If the method 200 determines the patient has only primary pulmonary disorder at block 202, then the method 200 proceeds to table 500, 550 illustrated in FIG. 38. If the method 200 determines the patient has only LHF and RHF at block 202, then the method 200 proceeds to table 600, 650 illustrated in FIG. 39. If the method 200 determines the patient has only LHF and primary pulmonary disorder at block 202, then the method 200 proceeds to table 700, 750 illustrated in FIG. 40. If the method 200 determines the patient has only RHF and primary pulmonary disorder at block 202, then the method 200 proceeds to table 800, 850 illustrated in FIG. 41. And, if the method 200 determines the patient has LHF, RHF, and primary pulmonary disorder at block 202, then the method 200 proceeds to table 900, 950 illustrated in FIG. 42. Once the appropriate table is referenced, the method 200 determines a treatment regimen for the patient based on the recommended treatment regimen from the table using the sensed LAP and/or the sensed RAP (block 218). In at least some embodiments, the monitoring system 72 may reference the appropriate table of the tables illustrated in FIGs. 36-42 and instruct (via a notification and/or other communication) the patient and/or medical professional to follow the treatment regimen proposed by the appropriate table. Once the treatment regimen is determined, administered, and/or communicated at block 218, the method 200 may return to block 210 to sense the LAP and/or RAP and continue through method 200 to determine whether the treatment regimen is effective. In at least some embodiments treatments may include, but are not limited to, diagnosis, medication titrations, advanced therapy, IV medications, lifestyle changes, intra-atrial shunts, valve repair/replace, ICDs, CRTs, and ablation. Exemplary medication titrations may include, but are not limited to, vasodilators, diuretics, pulmonary vasodilators, neurohormonal antagonists, beta blockers, and inotropes. Exemplary advanced therapies may include, but are not limited to, implanting a ventricular assist device (VAD), a transplant, or both. Exemplary lifestyle changes may include, but are not limited to, a change in diet, increased activity, or both.

The method 200 may be adjusted to be more specific in terms of how much a pharmacological, or medication regimen needs to be increased or reduced, which can be varied based on how much the LAP and RAP are above or below the baseline levels. This may be done by implementing another table or set of guidelines within the tables illustrated in FIGs. 36-42 that indicates the amount of medication to be administered (e.g., so that a treatment dosage regimen may be adjusted for a patient without requiring direct medical service provider intervention). Furthermore, to inform the patient on which medication to choose and its dosage, the type of medication (e.g. diuretic or vasodilator) that needs to be administered and the dosage thereof can be displayed on, for example, the screen of a computer or a display of a smart device used by the patient.

The measurement data and associated monitoring and treatment methodology is not necessarily limited to LAP and RAP measurements. In some examples, additional or alternative locations (e.g., pulmonary arteries, ventricles, pulmonary veins, aorta, and others) and/or additional or alternative metrics (e.g., temperature and/or oxygen saturation) may be utilized in implementing a monitoring and treatment method such as the method 200.

As explained above, the method 200 may be performed manually or may be partially or completely automated using any device capable of receiving and processing the measurement data from the measurement device 41. For example, the method 200 may be implemented entirely in the monitoring system 72 (e.g., such as a smart device), which performs all the comparisons, calculations, and determinations after receiving the LAP and RAP measurement data from the measurement system 41. In some examples, the method 200 may be implemented partially in the monitoring system 72 and partially in the communications relay 70 which may include a processing unit to receive the LAP and RAP measurement data from the sensors, determine whether the LAP and RAP are above/at/below normal level and decreasing/steady/increasing, then relay this information to the remote device 72 to perform the rest of the method. In yet another example, the subcutaneous implant 83 may be programmed to perform a portion or the entirety of the method 200.

In still further examples, the method 200 may be implemented in a device with a user interface allowing the patient to administer medications according to the results of the method 200. The method 200 may also be implemented in the medical service providers' electronic health record (EHR) or electronic medical record (EMR) systems which keep track of the necessary records of each patient. As such, the EHR or EMR systems may use local or remote database to access, among other things, the patient's history of LHF or RHF and whether the medical service providers have deemed the patient to be at a risk of such failure. The resulting data from the method 200 may be displayed on a dashboard of the user interface with multiple options for the user (e.g. patient and medical service providers), which may include: LAP and RAP averages, trend arrows, line graphs over time, and waveforms, as well as a history of the medications taken by the patient, etc. The dashboard may also be configured such that the user can first pull up the most meaningful information, such as the averages and trends, and a more detailed analysis can then be displayed, such as the waveforms. This may be implemented by organizing the multiple options in a hierarchical manner based on the importance of each option. In one example, this hierarchical order of the options is customizable according to the user's preference, such that the most preferred information can be pulled up first.

Referring to FIG. 36, an exemplary diagnostic regimen lookup table 300 and an exemplary treatment regimen lookup table 350 are illustrated for a patient diagnosed with the condition of LHF. In particular, the table 300 has three columns 302, 304, 306 and three rows 308, 310, 312 and each cell illustrates a pathology of the patient based on corresponding sensed heart pressure measurements for the patient. Similarly, the table 350 has three columns 352, 354, 356 and three rows 358, 360, 362 and each cell illustrates a treatment regimen for the patient based on corresponding sensed pressure measurements and the identified pathology in table 300. That is, columns 302, 352 pertain to the RAP sensed at block 210 (of FIG. 35) being lower than the baseline RAP determined at block 206 (of FIG. 35) by a threshold. Columns 304, 354 pertain to the RAP sensed at block 210 (of FIG. 35) being within a threshold of the baseline RAP determined at block 206 (of FIG. 35). Columns 306, 356 pertains to the RAP sensed at block 210 (of FIG. 35) being greater than the baseline RAP determined at block 206 (of FIG. 35) by a threshold. Rows 308, 358 pertain to the LAP sensed at block 210 (of FIG. 35) being higher than the baseline LAP determined at block 206 (of FIG. 35) by a threshold. Rows 310, 360 pertain to the LAP sensed at block 210 (of FIG. 35) being within a threshold of the baseline LAP determined at block 206 (of FIG. 35). And, rows 312, 362 pertain to the LAP sensed at block 210 (of FIG. 35) being less than the baseline LAP determined at block 206 (of FIG. 35) by a threshold.

Referring to columns 302, 352, the sensed RAP is lower than the baseline RAP by a threshold. In the event the sensed LAP is higher than the baseline LAP by a threshold, which corresponds to rows 308, 358, then it is likely the systemic vascular resistance (SVR) of the patient has increased so the treatment regimen for the patient is to increase the vasodilator dosing regimen of the patient according to table 350. As another example and still referring to columns 302, 352, if the sensed LAP is within a threshold of the baseline LAP, which corresponds to rows 310, 360, then it is likely the SVR of the patient has increased and the intravascular volume of the patient has decreased, so the vasodilator dosing regimen of the patient is increased while the diuretic dosing regimen of the patient is decreased. As even another example and still referring to columns 302, 352, if the sensed LAP is lower than the baseline LAP by a threshold, which corresponds to rows 312, 362, then it is likely the intravascular volume of the patient has decreased, and the corresponding treatment regimen indicated in table 350 is to decrease the diuretic dosing regimen of the patient.

Referring to columns 304, 354, the sensed RAP is within a threshold of the baseline RAP. In the event the sensed LAP is higher than the baseline LAP by a threshold, then it is likely the SVR of the patient has increased and the intravascular volume of the patient has increased. In this case, the vasodilator dosing regimen of the patient is increased, and the diuretic dosing regimen of the patient is increased. As another example and still referring to columns 304, 354, if the sensed LAP is within a threshold of the baseline LAP, then it is likely the dosing regimen of the patient is effective, and the current treatment regimen is maintained. As even another example and still referring to columns 304, 354, if the sensed LAP is lower than the baseline LAP by a threshold, then it is likely the pulmonary vascular resistance (PVR) of the patient has increased, or the patient is experiencing RHF and the intravascular volume of the patient has decreased. In this case, the monitoring system 72 may suggest the patient visit a medical professional for further testing and/or diagnosis.

Referring to columns 306, 356, the sensed RAP is greater than the baseline RAP by a threshold. In the event the sensed LAP is higher than the baseline LAP by a threshold, then it is likely the intravascular volume of the patient has increased, so the diuretic dosing regimen of the patient is increased. As another example and still referring to columns 306, 356, if the sensed LAP is within a threshold of the baseline LAP, then the patient is likely experiencing RHF and increased intravascular volume, or the PVR of the patient has increased and the intravascular volume of the patient has increased. In this case, the monitoring system 72 may suggest the patient visit a medical professional for further testing and/or diagnosis. Additionally, or alternatively, the diagnosis may be performed automatically by the monitoring system 72. As even another example and still referring to columns 306, 356, if the sensed LAP is lower than the baseline LAP by a threshold, then it is likely the patient is experiencing RHF. In this case, the monitoring system 72 may suggest the patient visit a medical professional for further testing and/or diagnosis. Additionally, or alternatively, the diagnosis may be performed automatically by the monitoring system 72. For any of these dosing regimen changes, the monitoring system 72 may send a notification and/or send corresponding instructions to a therapy device.

Referring to FIG. 37, an exemplary diagnostic regimen lookup table 400 and an exemplary treatment regimen lookup table 450 are illustrated for a patient diagnosed with the condition of RHF. In particular, the table 400 has three columns 402, 404, 406 and three rows 408, 410, 412 and each cell illustrates a pathology of the patient based on corresponding sensed heart pressure measurements for the patient. Similarly, the table 450 has three columns 452, 454, 456 and three rows 458, 460, 462 and each cell illustrates a treatment regimen for the patient based on corresponding sensed pressure measurements and the identified pathology in table 400. That is, columns 402, 452 pertain to the RAP sensed at block 210 (of FIG. 35) being lower than the baseline RAP determined at block 206 (of FIG. 35) by a threshold. Columns 404, 454 pertain to the RAP sensed at block 210 (of FIG. 35) being within a threshold of the baseline RAP determined at block 206 (of FIG. 35). Columns 406, 456 pertains to the RAP sensed at block 210 (of FIG. 35) being greater than the baseline RAP determined at block 206 (of FIG. 35) by a threshold. Rows 408, 458 pertain to the LAP sensed at block 210 (of FIG. 35) being higher than the baseline LAP determined at block 206 (of FIG. 35) by a threshold. Rows 410, 460 pertain to the LAP sensed at block 210 (of FIG. 35) being within a threshold of the baseline LAP determined at block 206 (of FIG. 35). And, rows 412, 462 pertain to the LAP sensed at block 210 (of FIG. 35) being less than the baseline LAP determined at block 206 (of FIG. 35) by a threshold.

Referring to columns 402, 452, the sensed RAP is lower than the baseline RAP by a threshold. In the event the sensed LAP is higher than the baseline LAP by a threshold, which corresponds to rows 408, 458, then it is likely the patient is experiencing LHF. In this case, the monitoring system 72 may suggest the patient visit a medical professional for further testing and/or diagnosis. Additionally, or alternatively, the diagnosis may be performed automatically by the monitoring system 72. As another example and still referring to columns 402, 452, if the sensed LAP is within a threshold of the baseline LAP, which corresponds to rows 410, 460, then it is likely the patient is experiencing LHF and the intravascular volume of the patient has decreased. In this case, the monitoring system 72 may suggest the patient visit a medical professional for further testing and/or diagnosis. Additionally, or alternatively, the diagnosis may be performed automatically by the monitoring system 72. As even another example and still referring to columns 402, 452, if the sensed LAP is lower than the baseline LAP by a threshold, which corresponds to rows 412, 462, then it is likely the intravascular volume of the patient has decreased, and the corresponding treatment regimen is to decrease the diuretic dosing regimen of the patient

Referring to columns 404, 454, the sensed RAP is within a threshold of the baseline RAP. In the event the sensed LAP is higher than the baseline LAP by a threshold, then it is likely the patient is experiencing LHF and increased intravascular volume. In this case, the monitoring system 72 may suggest the patient visit a medical professional for further testing and/or diagnosis. Additionally, or alternatively, the diagnosis may be performed automatically by the monitoring system 72. As another example and still referring to columns 404, 454, if the sensed LAP is within a threshold of the baseline LAP, then it is likely the dosing regimen of the patient is effective, and the current treatment regimen is maintained. As even another example and still referring to columns 404, 454, if the sensed LAP is lower than the baseline LAP by a threshold, then it is likely the patient's RHF is getting worse and the intravascular volume of the patient has decreased. In this case, the corresponding treatment regimen is to increase the pulmonary vasodilators treatment regimen and decrease the diuretic treatment regimen.

Referring to columns 406, 456, the sensed RAP is greater than the baseline RAP by a threshold. In the event the sensed LAP is higher than the baseline LAP by a threshold, then it is likely the intravascular volume of the patient has increased so the corresponding treatment regimen indicated in table 450 is to increase the diuretic dosing regimen of the patient. As another example and still referring to columns 406, 456, if the sensed LAP is within a threshold of the baseline LAP, then the patient's RHF is likely worsening and the intravascular volume of the patient has increased. In this case, the corresponding dosing regimen indicated in table 450 is to increase pulmonary vasodilators and increase diuretics. As even another example and still referring to columns 406, 456, if the sensed LAP is lower than the baseline LAP by a threshold, then it is likely the patient's RHF is worsening. In this case, the corresponding treatment regimen indicated in table 450 is to increase the pulmonary vasodilators. For any of these dosing regimen changes, the monitoring system 72 may send a notification and/or send corresponding instructions to a therapy device.

Referring to FIG. 38, an exemplary diagnostic regimen lookup table 500 and an exemplary treatment regimen lookup table 550 are illustrated for a patient diagnosed with the condition of primary pulmonary disorder. In particular, the table 500 has three columns 502, 504, 506 and three rows 508, 510, 512 and each cell illustrates a pathology of the patient based on corresponding sensed heart pressure measurements for the patient. Similarly, the table 550 has three columns 552, 554, 556 and three rows 558, 560, 562 and each cell illustrates a treatment regimen for the patient based on corresponding sensed pressure measurements and the identified pathology in Table 500. That is, columns 502, 552 pertain to the RAP sensed at block 210 (of FIG. 35) being lower than the baseline RAP determined at block 206 (of FIG. 35) by a threshold. Columns 504, 554 pertain to the RAP sensed at block 210 (of FIG. 35) being within a threshold of the baseline RAP determined at block 206 (of FIG. 35). Columns 506, 556 pertains to the RAP sensed at block 210 (of FIG. 35) being greater than the baseline RAP determined at block 206 (of FIG. 35) by a threshold. Rows 508, 558 pertain to the LAP sensed at block 210 (of FIG. 35) being higher than the baseline LAP determined at block 206 (of FIG. 35) by a threshold. Rows 510, 560 pertain to the LAP sensed at block 210 (of FIG. 35) being within a threshold of the baseline LAP determined at block 206 (of FIG. 35). And, rows 512, 562 pertain to the LAP sensed at block 210 (of FIG. 35) being less than the baseline LAP determined at block 206 (of FIG. 35) by a threshold.

Referring to columns 502, 552, the sensed RAP is lower than the baseline RAP by a threshold. In the event the sensed LAP is higher than the baseline LAP by a threshold, which corresponds to rows 508, 558, then it is likely the patient is experiencing LHF. In this case, the monitoring system 72 may suggest the patient visit a medical professional for further testing and/or diagnosis. Additionally, or alternatively, the diagnosis may be performed automatically by the monitoring system 72. As another example and still referring to columns 502, 552, if the sensed LAP is within a threshold of the baseline LAP, which corresponds to rows 510, 560, then it is likely the patient is experiencing LHF and the intravascular volume of the patient has decreased. In this case, the monitoring system 72 may suggest the patient visit a medical professional for further testing and/or diagnosis. Additionally, or alternatively, the diagnosis may be performed automatically by the monitoring system 72. As even another example and still referring to columns 502, 552, if the sensed LAP is lower than the baseline LAP by a threshold, which corresponds to rows 512, 562, then it is likely the intravascular volume of the patient has decreased, and the corresponding treatment regimen is to decrease the diuretic dosing regimen of the patient.

Referring to columns 504, 554, the sensed RAP is within a threshold of the baseline RAP. In the event the sensed LAP is higher than the baseline LAP by a threshold, then it is likely the patient is experiencing LHF and increased intravascular volume. In this case, the monitoring system 72 may suggest the patient visit a medical professional for further testing and/or diagnosis. Additionally, or alternatively, the diagnosis may be performed automatically by the monitoring system 72. As another example and still referring to columns 504, 554, if the sensed LAP is within a threshold of the baseline LAP, then it is likely the dosing regimen of the patient is effective, and the current treatment regimen is maintained. As even another example and still referring to columns 504, 554, if the sensed LAP is lower than the baseline LAP by a threshold, then it is likely the patient's PVR is worsening. In this case, the corresponding treatment regimen is to increase the pulmonary vasodilators treatment regimen.

Referring to columns 506, 556, the sensed RAP is greater than the baseline RAP by a threshold. In the event the sensed LAP is higher than the baseline LAP by a threshold, then it is likely the intravascular volume of the patient has increased so the corresponding treatment regimen indicated in table 550 is to increase the diuretic dosing regimen of the patient. As another example and still referring to columns 506, 556, if the sensed LAP is within a threshold of the baseline LAP, then the patient is experiencing RHF and the intravascular volume of the patient has increased, or the patients PVR is worsening and the intravascular volume of the patient has increased. In this case, the corresponding dosing regimen indicated in table 550 is to increase pulmonary vasodilators and increase diuretics. As even another example and still referring to columns 506, 556, if the sensed LAP is lower than the baseline LAP by a threshold, then it is likely the patient is experiencing RHF. In this case, the monitoring system 72 may suggest the patient visit a medical professional for further testing and/or diagnosis. Additionally, or alternatively, the diagnosis may be performed automatically by the monitoring system 72. For any of these dosing regimen changes, the monitoring system 72 may send a notification and/or send corresponding instructions to a therapy device.

Referring to FIG. 39, an exemplary diagnostic regimen lookup table 600 and an exemplary treatment regimen lookup table 650 are illustrated for a patient diagnosed with the condition of LHF and RHF. In particular, the table 600 has three columns 602, 604, 606 and three rows 608, 610, 612 and each cell illustrates a pathology of the patient based on corresponding sensed heart pressure measurements for the patient. Similarly, the table 650 has three columns 652, 654, 656 and three rows 658, 660, 662 and each cell illustrates a treatment regimen for the patient based on corresponding sensed pressure measurements and the identified pathology in Table 600. That is, columns 602, 652 pertain to the RAP sensed at block 210 (of FIG. 35) being lower than the baseline RAP determined at block 206 (of FIG. 35) by a threshold. Columns 604, 654 pertain to the RAP sensed at block 210 (of FIG. 35) being within a threshold of the baseline RAP determined at block 206 (of FIG. 35). Columns 606, 656 pertains to the RAP sensed at block 210 (of FIG. 35) being greater than the baseline RAP determined at block 206 (of FIG. 35) by a threshold. Rows 608, 658 pertain to the LAP sensed at block 210 (of FIG. 35) being higher than the baseline LAP determined at block 206 (of FIG. 35) by a threshold. Rows 610, 660 pertain to the LAP sensed at block 210 (of FIG. 35) being within a threshold of the baseline LAP determined at block 206 (of FIG. 35). And, rows 612, 662 pertain to the LAP sensed at block 210 (of FIG. 35) being less than the baseline LAP determined at block 206 (of FIG. 35) by a threshold.

Referring to columns 602, 652, the sensed RAP is lower than the baseline RAP by a threshold. In the event the sensed LAP is higher than the baseline LAP by a threshold, which corresponds to rows 608, 658, then it is likely the SVR of the patient has increased so the treatment regimen for the patient is to increase the vasodilator dosing regimen of the patient, as indicated in table 650. As another example and still referring to columns 602, 652, if the sensed LAP is within a threshold of the baseline LAP, which corresponds to rows 610, 660, then it is likely the SVR of the patient has increased and the intravascular volume of the patient has decreased, so the corresponding treatment regimen indicated in table 650 is to increase the vasodilator dosing regimen of the patient while decreasing the diuretic dosing regimen of the patient. As even another example and still referring to columns 602, 652, if the sensed LAP is lower than the baseline LAP by a threshold, which corresponds to rows 612, 662, then it is likely the intravascular volume of the patient has decreased, and the corresponding treatment regimen indicated in table 650 is to decrease the diuretic dosing regimen of the patient.

Referring to columns 604, 654, the sensed RAP is within a threshold of the baseline RAP. In the event the sensed LAP is higher than the baseline LAP by a threshold, then it is likely the SVR of the patient has increased and the intravascular volume of the patient has increased. In this case, the vasodilator dosing regimen of the patient is increased, and the diuretic dosing regimen of the patient is increased. As another example and still referring to columns 604, 654, if the sensed LAP is within a threshold of the baseline LAP, then it is likely the dosing regimen of the patient is effective, and the current treatment regimen is maintained. As even another example and still referring to columns 604, 654, if the sensed LAP is lower than the baseline LAP by a threshold, then it is likely the patient's RHF is worsening and the intravascular volume of the patient has decreased. In this case, the treatment regimen indicated in table 650 is to increase the pulmonary vasodilators dosing regimen and decrease the diuretic dosing regimen.

Referring to columns 606, 656, the sensed RAP is greater than the baseline RAP by a threshold. In the event the sensed LAP is higher than the baseline LAP by a threshold, then it is likely the intravascular volume of the patient has increased so the corresponding treatment regimen indicated in table 650 is to increase the diuretic dosing regimen of the patient. As another example and still referring to columns 606, 656, if the sensed LAP is within a threshold of the baseline LAP, then the patient's RHF is likely worsening and the intravascular volume of the patient has increased. In this case, the corresponding dosing regimen indicated in table 650 is to increase pulmonary vasodilators and increase diuretics. As even another example and still referring to columns 606, 656, if the sensed LAP is lower than the baseline LAP by a threshold, then it is likely the patient's RHF is worsening. In this case, the corresponding treatment regimen indicated in table 650 is to increase the pulmonary vasodilators. For any of these dosing regimen changes, the monitoring system 72 may send a notification and/or send corresponding instructions to a therapy device.

Referring to FIG. 40, an exemplary diagnostic regimen lookup table 700 and an exemplary treatment regimen lookup table 750 are illustrated for a patient diagnosed with the condition of LHF and primary pulmonary disorder. In particular, the table 700 has three columns 702, 704, 706 and three rows 708, 710, 712 and each cell illustrates a pathology of the patient based on corresponding sensed heart pressure measurements for the patient. Similarly, the table 750 has three columns 752, 754, 756 and three rows 758, 760, 762 and each cell illustrates a treatment regimen for the patient based on corresponding sensed pressure measurements and the identified pathology in Table 700. That is, columns 702, 752 pertain to the RAP sensed at block 210 (of FIG. 35) being lower than the baseline RAP determined at block 206 (of FIG. 35) by a threshold. Columns 704, 754 pertain to the RAP sensed at block 210 (of FIG. 35) being within a threshold of the baseline RAP determined at block 206 (of FIG. 35). Columns 706, 756 pertains to the RAP sensed at block 210 (of FIG. 35) being greater than the baseline RAP determined at block 206 (of FIG. 35) by a threshold. Rows 708, 758 pertain to the LAP sensed at block 210 (of FIG. 35) being higher than the baseline LAP determined at block 206 (of FIG. 35) by a threshold. Rows 710, 760 pertain to the LAP sensed at block 210 (of FIG. 35) being within a threshold of the baseline LAP determined at block 206 (of FIG. 35). And, rows 712, 762 pertain to the LAP sensed at block 210 (of FIG. 35) being less than the baseline LAP determined at block 206 (of FIG. 35) by a threshold.

Referring to columns 702, 752, the sensed RAP is lower than the baseline RAP by a threshold. In the event the sensed LAP is higher than the baseline LAP by a threshold, which corresponds to rows 708, 758, then it is likely the SVR of the patient has increased so the treatment regimen for the patient is to increase the vasodilator dosing regimen of the patient, as indicated in table 750. As another example and still referring to columns 702, 752, if the sensed LAP is within a threshold of the baseline LAP, which corresponds to rows 710, 760, then it is likely the SVR of the patient has increased and the intravascular volume of the patient has decreased, so the corresponding treatment regimen indicated in table 750 is to increase the vasodilator dosing regimen of the patient while decreasing the diuretic dosing regimen of the patient. As even another example and still referring to columns 702, 752, if the sensed LAP is lower than the baseline LAP by a threshold, which corresponds to rows 712, 762, then it is likely the intravascular volume of the patient has decreased, and the corresponding treatment regimen indicated in table 750 is to decrease the diuretic dosing regimen of the patient.

Referring to columns 704, 754, the sensed RAP is within a threshold of the baseline RAP. In the event the sensed LAP is higher than the baseline LAP by a threshold, then it is likely the SVR of the patient has increased and the intravascular volume of the patient has increased. In this case, the corresponding treatment regimen indicated in table 750 is to increase the vasodilator dosing regimen for the patient and increase the diuretic dosing regimen of the patient. As another example and still referring to columns 704, 754, if the sensed LAP is within a threshold of the baseline LAP, then it is likely the dosing regimen of the patient is effective, and the current treatment regimen is maintained. As even another example and still referring to columns 704, 754, if the sensed LAP is lower than the baseline LAP by a threshold, then it is likely the patient's PVR is worsening. In this case, the treatment regimen indicated in table 750 is to increase the pulmonary vasodilators dosing regimen for the patient.

Referring to columns 706, 756, the sensed RAP is greater than the baseline RAP by a threshold. In the event the sensed LAP is higher than the baseline LAP by a threshold, then it is likely the intravascular volume of the patient has increased so the corresponding treatment regimen indicated in table 750 is to increase the diuretic dosing regimen of the patient. As another example and still referring to columns 706, 756, if the sensed LAP is within a threshold of the baseline LAP, then the patient's PVR is likely worsening and the intravascular volume of the patient has increased. In this case, the corresponding dosing regimen indicated in table 750 is to increase pulmonary vasodilators and increase diuretics. As even another example and still referring to columns 706, 756, if the sensed LAP is lower than the baseline LAP by a threshold, then it is likely the patient is experiencing RHF. In this case, the monitoring system 72 may suggest the patient visit a medical professional for further testing and/or diagnosis. Additionally, or alternatively, the diagnosis may be performed automatically by the monitoring system 72. For any of these dosing regimen changes, the monitoring system 72 may send a notification and/or send corresponding instructions to a therapy device.

Referring to FIG. 41, an exemplary diagnostic regimen lookup table 800 and an exemplary treatment regimen lookup table 850 are illustrated for a patient diagnosed with the condition of RHF and primary pulmonary disorder. In particular, the table 800 has three columns 802, 804, 806 and three rows 808, 810, 812 and each cell illustrates a pathology of the patient based on corresponding sensed heart pressure measurements for the patient. Similarly, the table 850 has three columns 852, 854, 856 and three rows 858, 860, 862 and each cell illustrates a treatment regimen for the patient based on corresponding sensed pressure measurements and the identified pathology in Table 800. That is, columns 802, 852 pertain to the RAP sensed at block 210 (of FIG. 35) being lower than the baseline RAP determined at block 206 (of FIG. 35) by a threshold. Columns 804, 854 pertain to the RAP sensed at block 210 (of FIG. 35) being within a threshold of the baseline RAP determined at block 206 (of FIG. 35). Columns 806, 856 pertains to the RAP sensed at block 210 (of FIG. 35) being greater than the baseline RAP determined at block 206 (of FIG. 35) by a threshold. Rows 808, 858 pertain to the LAP sensed at block 210 (of FIG. 35) being higher than the baseline LAP determined at block 206 (of FIG. 35) by a threshold. Rows 810, 860 pertain to the LAP sensed at block 210 (of FIG. 35) being within a threshold of the baseline LAP determined at block 206 (of FIG. 35). And, rows 812, 862 pertain to the LAP sensed at block 210 (of FIG. 35) being less than the baseline LAP determined at block 206 (of FIG. 35) by a threshold.

Referring to columns 802, 852, the sensed RAP is lower than the baseline RAP by a threshold. In the event the sensed LAP is higher than the baseline LAP by a threshold, which corresponds to rows 808, 858, then it is likely the patient is experiencing LHF. In this case, the monitoring system 72 may suggest the patient visit a medical professional for further testing and/or diagnosis. Additionally, or alternatively, the diagnosis may be performed automatically by the monitoring system 72. As another example and still referring to columns 802, 852, if the sensed LAP is within a threshold of the baseline LAP, which corresponds to rows 810, 860, then it is likely the patient is experiencing LHF and the intravascular volume of the patient has decreased. In this case, the monitoring system 72 may suggest the patient visit a medical professional for further testing and/or diagnosis. Additionally, or alternatively, the diagnosis may be performed automatically by the monitoring system 72. As even another example and still referring to columns 802, 852, if the sensed LAP is lower than the baseline LAP by a threshold, which corresponds to rows 812, 862, then it is likely the intravascular volume of the patient has decreased, and the corresponding treatment regimen is to decrease the diuretic dosing regimen of the patient.

Referring to columns 804, 854, the sensed RAP is within a threshold of the baseline RAP. In the event the sensed LAP is higher than the baseline LAP by a threshold, then it is likely the patient is experiencing LHF and increased intravascular volume. In this case, the monitoring system 72 may suggest the patient visit a medical professional for further testing and/or diagnosis. Additionally, or alternatively, the diagnosis may be performed automatically by the monitoring system 72. As another example and still referring to columns 804, 854, if the sensed LAP is within a threshold of the baseline LAP, then it is likely the dosing regimen of the patient is effective, and the current treatment regimen is maintained. As even another example and still referring to columns 804, 854, if the sensed LAP is lower than the baseline LAP by a threshold, then it is likely the patient's PVR is worsening or the patient's RHF is worsening and the intravascular volume of the patient has increased. In this case, the corresponding treatment regimen illustrated in table 850 is to increase the pulmonary vasodilators treatment regimen and decrease the diuretic treatment regimen for the patient.

Referring to columns 806, 856, the sensed RAP is greater than the baseline RAP by a threshold. In the event the sensed LAP is higher than the baseline LAP by a threshold, then it is likely the intravascular volume of the patient has increased so the corresponding treatment regimen indicated in table 850 is to increase the diuretic dosing regimen of the patient. As another example and still referring to columns 806, 856, if the sensed LAP is within a threshold of the baseline LAP, then the patient's RHF is likely worsening and the intravascular volume of the patient has increased or the patient's PVR is worsening and the intravascular volume of the patient has increased. In this case, the corresponding dosing regimen indicated in table 850 is to increase pulmonary vasodilators and increase diuretics. As even another example and still referring to columns 806, 856, if the sensed LAP is lower than the baseline LAP by a threshold, then it is likely the patient's RHF is worsening. In this case, the corresponding treatment regimen indicated in table 850 is to increase the pulmonary vasodilators. For any of these dosing regimen changes, the monitoring system 72 may send a notification and/or send corresponding instructions to a therapy device.

Referring to FIG. 42, an exemplary diagnostic regimen lookup table 900 and an exemplary treatment regimen lookup table 950 for a patient diagnosed with the condition of LHF, RHF, and primary pulmonary disorder are illustrated. In particular, the table 900 has three columns 902, 904, 906 and three rows 908, 910, 912 and each cell illustrates a pathology of the patient based on corresponding sensed heart pressure measurements for the patient. Similarly, the table 950 has three columns 952, 954, 956 and three rows 958, 960, 962 and each cell illustrates a treatment regimen for the patient based on corresponding sensed pressure measurements and the identified pathology in Table 900. That is, columns 902, 952 pertain to the RAP sensed at block 210 (of FIG. 35) being lower than the baseline RAP determined at block 206 (of FIG. 35) by a threshold. Columns 904, 954 pertain to the RAP sensed at block 210 (of FIG. 35) being within a threshold of the baseline RAP determined at block 206 (of FIG. 35). Columns 906, 956 pertains to the RAP sensed at block 210 (of FIG. 35) being greater than the baseline RAP determined at block 206 (of FIG. 35) by a threshold. Rows 908, 958 pertain to the LAP sensed at block 210 (of FIG. 35) being higher than the baseline LAP determined at block 206 (of FIG. 35) by a threshold. Rows 910, 960 pertain to the LAP sensed at block 210 (of FIG. 35) being within a threshold of the baseline LAP determined at block 206 (of FIG. 35). And, rows 912, 962 pertain to the LAP sensed at block 210 (of FIG. 35) being less than the baseline LAP determined at block 206 (of FIG. 35) by a threshold.

Referring to columns 902, 952, the sensed RAP is lower than the baseline RAP by a threshold. In the event the sensed LAP is higher than the baseline LAP by a threshold, which corresponds to rows 908, 958, then it is likely the SVR of the patient has increased so the treatment regimen for the patient is to increase the vasodilator dosing regimen of the patient, as indicated in table 950. As another example and still referring to columns 902, 952, if the sensed LAP is within a threshold of the baseline LAP, which corresponds to rows 910, 960, then it is likely the SVR of the patient has increased and the intravascular volume of the patient has decreased, so the corresponding treatment regimen indicated in table 950 is to increase the vasodilator dosing regimen of the patient while decreasing the diuretic dosing regimen of the patient. As even another example and still referring to columns 902, 952, if the sensed LAP is lower than the baseline LAP by a threshold, which corresponds to rows 912, 962, then it is likely the intravascular volume of the patient has decreased, and the corresponding treatment regimen indicated in table 950 is to decrease the diuretic dosing regimen of the patient.

Referring to columns 904, 954, the sensed RAP is within a threshold of the baseline RAP. In the event the sensed LAP is higher than the baseline LAP by a threshold, then it is likely the SVR of the patient has increased and the intravascular volume of the patient has increased. In this case, the vasodilator dosing regimen of the patient is increased, and the diuretic dosing regimen of the patient is increased. As another example and still referring to columns 904, 954, if the sensed LAP is within a threshold of the baseline LAP, then it is likely the dosing regimen of the patient is effective, and the current treatment regimen is maintained. As even another example and still referring to columns 904, 954, if the sensed LAP is lower than the baseline LAP by a threshold, then it is likely the patient's PVR has increased, or the patient's RHF is worsening and the intravascular volume of the patient has decreased. In this case, the treatment regimen indicated in table 950 is to increase the pulmonary vasodilators dosing regimen and decrease the diuretic dosing regimen.

Referring to columns 906, 956, the sensed RAP is greater than the baseline RAP by a threshold. In the event the sensed LAP is higher than the baseline LAP by a threshold, then it is likely the intravascular volume of the patient has increased so the corresponding treatment regimen indicated in table 950 is to increase the diuretic dosing regimen of the patient. As another example and still referring to columns 906, 956, if the sensed LAP is within a threshold of the baseline LAP, then the patient's RHF is likely worsening and the intravascular volume of the patient has increased, or the patient's PVR has increased and the intravascular volume of the patient has increased. In this case, the corresponding dosing regimen indicated in table 950 is to increase pulmonary vasodilators and increase diuretics. As even another example and still referring to columns 906, 956, if the sensed LAP is lower than the baseline LAP by a threshold, then it is likely the patient's RHF is worsening. In this case, the corresponding treatment regimen indicated in table 950 is to increase the pulmonary vasodilators. For any of these dosing regimen changes, the monitoring system 72 may send a notification and/or send corresponding instructions to a therapy device.

In some embodiments, the method 200 (and/or algorithm 99) may incorporate additional metrics such as systemic blood pressure and heart rate to determine the addition of other medications beyond diuretics, vasodilators, and pulmonary vasodilators to address a rise in pressure. For example, an increase in heart rate may determine the need for an increase in dosage of beta blockers instead of vasodilators in order to reduce a high LAP pressure. As another example, a very low blood pressure combined with high LAP and high RAP may determine the need for inotropes instead of diuretics. Additionally, in at least some embodiment, the method 200 (and/or algorithm 99) may be used to indicate the need for treatments beyond medications titrations including lifestyle changes (diet, activity), advanced therapy (VADs, transplant), or therapeutic interventions (intra-atrial shunts, CRTs, ICDs, valve repair/replacement, ablations, etc.)

Persons skilled in the art will readily appreciate that various aspects of the present disclosure can be realized by any number of methods and apparatus configured to perform the intended functions. It should also be noted that the accompanying drawing figures referred to herein are not necessarily drawn to scale but may be exaggerated to illustrate various aspects of the present disclosure, and in that regard, the drawing figures should not be construed as limiting.

The invention of this application has been described above both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims.

## Claims

1. A medical system (41, 42) for determining a treatment regimen for a patient with a condition, the system (41, 42) comprising:
at least one sensor (10, 11) configured to sense right atrial pressure and left atrial pressure; and
a receiver configured to receive measurement data corresponding to the sensed right atrial pressure and the sensed left atrial pressure, to receive the condition of the patient, and output to a display device the received measurement data:
a memory unit configured to store the received measurement data and the condition of the patient; and
a processor configured to:
compare the sensed right atrial pressure to a baseline right atrial pressure to determine a first comparison;
compare the sensed left atrial pressure to a baseline left atrial pressure to determine a second comparison; and
determine the treatment regimen for the patient based on the comparisons and the condition of the patient,
wherein the condition is at least one selected from the group of: left heart failure, right heart failure, and primary pulmonary disorder.

2. The medical system (41, 42) of claim 1, wherein to determine the treatment regimen of the patient, the processor is configured to: compare the sensed right atrial pressure and the sensed left atrial pressure.

3. The medical system (41, 42) of any one of claims 1 or 2, wherein to determine the treatment regimen for the patient, the processor is configured to provide a notification to increase the dosage of the treatment regimen.

4. The medical system (41, 42) of any one of claims 1 or 2, wherein to determine the treatment regimen for the patient, the processor is configured to provide a notification to decrease the dosage of the treatment regimen.

5. The medical system (41, 42) of any one of claims 1-4, wherein the processor is incorporated into an implantable medical device.

6. The medical system (41, 42) of any one of claims 1-4, wherein the processor is incorporated into a device located external to the patient.

7. The medical system (41, 42) of any one of claims 1-6, wherein to determine the treatment regimen of the patient, the processor is configured to provide a notification to increase at least one treatment selected from the following group of treatments: vasodilators, diuretics, pulmonary vasodilators, neurohormonal antagonists, beta blockers, and inotropes.

8. The medical system (41, 42) of any one of claims 1-6, wherein to determine the treatment regimen of the patient, the processor is configured to provide a notification to decrease at least one treatment selected from the following group of treatments: vasodilators, diuretics pulmonary vasodilators, neurohormonal antagonists, beta blockers, and inotropes.

9. A computer-implemented method (200) for determining a treatment regimen for a patient with a condition, the method comprising:
receiving at least one measurement corresponding to a sensed right atrial pressure for the patient;
receiving at least one measurement corresponding to a sensed left atrial pressure for the patient;
receiving the condition of the patient;
comparing the sensed right atrial pressure to a baseline right atrial pressure to determine a first comparison;
comparing the sensed left atrial pressure to a baseline left atrial pressure to determine a second comparison;
determining the treatment regimen for the patient based on the first comparison, the second comparison, and the condition of the patient; and
outputting to a display device: the at least one measurement corresponding to the sensed right atrial pressure, the at least one measurement corresponding to the sensed left atrial pressure, and the determined treatment regimen,
wherein the condition of the patient is at least one selected from the group of: left heart failure, right heart failure, and primary pulmonary disorder.

10. The method (200) of claim 9, wherein determining the treatment regimen of the patient comprises comparing the sensed right atrial pressure and the sensed left atrial pressure.

11. The method (200) of any one of claims 9 or 10, wherein determining the treatment regimen for the patient comprises providing a notification to increase the dosage of the treatment regimen.

12. The method (200) of any one of claims 9 or 10, wherein determining the treatment regimen for the patient comprises providing a notification to decrease the dosage of the treatment regimen.

13. The method (200) of any one of claims 9-12, wherein determining the treatment regimen of the patient comprises providing a notification to increase at least one treatment selected from the following group of treatments: vasodilators, diuretics, pulmonary vasodilators, neurohormonal antagonists, beta blockers, and inotropes.

14. The method (200) of any one of claims 9-12, wherein determining the treatment regimen of the patient comprises providing a notification to decrease at least one treatment selected from the following group of treatments: vasodilators, diuretics, and pulmonary vasodilators, neurohormonal antagonists, beta blockers, and inotropes.

## Patentansprüche

1. Medizinisches System (41, 42) zum Bestimmen eines Behandlungsplans für einen Patienten mit einer Erkrankung, wobei das System (41, 42) umfasst:
mindestens einen Sensor (10, 11), der dazu konfiguriert ist, den Druck im rechten Vorhof und den Druck im linken Vorhof zu erfassen; und
einen Empfänger, der dazu konfiguriert ist, Messdaten zu empfangen, die dem erfassten Druck im rechten Vorhof und dem erfassten Druck im linken Vorhof entsprechen, die Erkrankung des Patienten zu empfangen und die empfangenen Messdaten an ein Anzeigegerät auszugeben:
eine Speichereinheit, die dazu konfiguriert ist, die empfangenen Messdaten und die Erkrankung des Patienten zu speichern; und
einen Prozessor, der konfiguriert ist zum:
Vergleichen des erfassten Druckes im rechten Vorhof mit einem Grundliniendruck im rechten Vorhof, um einen ersten Vergleich zu bestimmen;
Vergleichen des erfassten Druckes im linken Vorhof mit einem Grundliniendruck im linken Vorhof, um einen zweiten Vergleich zu bestimmen; und
Bestimmen des Behandlungsplans für den Patienten auf der Grundlage der Vergleiche und der Erkrankung des Patienten,
wobei die Erkrankung mindestens eine ist, die ausgewählt ist aus der Gruppe: Linksherzinsuffizienz, Rechtsherzinsuffizienz und primäre Lungenerkrankung.

2. Medizinisches System (41, 42) nach Anspruch 1, wobei zum Bestimmen des Behandlungsplans des Patienten der Prozessor dazu konfiguriert ist: den erfassten Druck im rechten Vorhof und den erfassten Druck im linken Vorhof zu vergleichen.

3. Medizinisches System (41, 42) nach einem der Ansprüche 1 oder 2, wobei zum Bestimmen des Behandlungsplans für den Patienten der Prozessor dazu konfiguriert ist, eine Benachrichtigung zum Erhöhen der Dosierung des Behandlungsplans bereitzustellen.

4. Medizinisches System (41, 42) nach einem der Ansprüche 1 oder 2, wobei zum Bestimmen des Behandlungsplans für den Patienten der Prozessor dazu konfiguriert ist, eine Benachrichtigung zum Verringern der Dosierung des Behandlungsplans bereitzustellen.

5. Medizinisches System (41, 42) nach einem der Ansprüche 1-4, wobei der Prozessor in ein implantierbares medizinisches Gerät integriert ist.

6. Medizinisches System (41, 42) nach einem der Ansprüche 1-4, wobei der Prozessor in ein Gerät integriert ist, das sich außerhalb des Patienten befindet.

7. Medizinisches System (41, 42) nach einem der Ansprüche 1-6, wobei zum Bestimmen des Behandlungsplans des Patienten der Prozessor dazu konfiguriert ist, eine Benachrichtigung zum Erhöhen mindestens einer Behandlung bereitzustellen, die aus der folgenden Gruppe von Behandlungen ausgewählt wird: Vasodilatatoren, Diuretika, pulmonale Vasodilatatoren, neurohormonelle Antagonisten, Betablocker und Inotropika.

8. Medizinisches System (41, 42) nach einem der Ansprüche 1-6, wobei zum Bestimmen des Behandlungsplans des Patienten der Prozessor dazu konfiguriert ist, eine Benachrichtigung zum Verringern mindestens einer Behandlung bereitzustellen, die aus der folgenden Gruppe von Behandlungen ausgewählt wird: Vasodilatatoren, Diuretika, pulmonale Vasodilatatoren, neurohormonelle Antagonisten, Betablocker und Inotropika.

9. Computerimplementiertes Verfahren (200) zum Bestimmen eines Behandlungsplans für einen Patienten mit einer Erkrankung, wobei das Verfahren umfasst:
Empfangen mindestens einer Messung, die einem erfassten Druck im rechten Vorhof für den Patienten entspricht;
Empfangen mindestens einer Messung, die einem erfassten Druck im linken Vorhof für den Patienten entspricht;
Empfangen der Erkrankung des Patienten;
Vergleichen des erfassten Druckes im rechten Vorhof mit einem Grundliniendruck im rechten Vorhof, um einen ersten Vergleich zu bestimmen;
Vergleichen des erfassten Druckes im linken Vorhof mit einem Grundliniendruck im linken Vorhof, um einen zweiten Vergleich zu bestimmen;
Bestimmen des Behandlungsplans für den Patienten auf der Grundlage des ersten Vergleichs, des zweiten Vergleichs und der Erkrankung des Patienten; und
Ausgeben an ein Anzeigegerät: der mindestens einen Messung, die dem erfassten Druck im rechten Vorhof entspricht, der mindestens einen Messung, die dem erfassten Druck im linken Vorhof entspricht, und des bestimmten Behandlungsplans,
wobei die Erkrankung des Patienten mindestens eine ist, die ausgewählt ist aus der Gruppe: Linksherzinsuffizienz, Rechtsherzinsuffizienz und primäre Lungenerkrankung.

10. Verfahren (200) nach Anspruch 9, wobei das Bestimmen des Behandlungsplans des Patienten das Vergleichen des erfassten Druckes im rechten Vorhof und des erfassten Druckes im linken Vorhof umfasst.

11. Verfahren (200) nach einem der Ansprüche 9 oder 10, wobei das Bestimmen des Behandlungsplans für den Patienten das Bereitstellen einer Benachrichtigung zum Erhöhen der Dosierung des Behandlungsplans umfasst.

12. Verfahren (200) nach einem der Ansprüche 9 oder 10, wobei das Bestimmen des Behandlungsplans für den Patienten das Bereitstellen einer Benachrichtigung zum Verringern der Dosierung des Behandlungsplans umfasst.

13. Verfahren (200) nach einem der Ansprüche 9-12, wobei das Bestimmen des Behandlungsplans des Patienten das Bereitstellen einer Benachrichtigung zum Erhöhen mindestens einer Behandlung umfasst, die aus der folgenden Gruppe von Behandlungen ausgewählt wird: Vasodilatatoren, Diuretika, pulmonale Vasodilatatoren, neurohormonelle Antagonisten, Betablocker und Inotropika.

14. Verfahren (200) nach einem der Ansprüche 9-12, wobei das Bestimmen des Behandlungsplans des Patienten das Bereitstellen einer Benachrichtigung zum Verringern mindestens einer Behandlung umfasst, die aus der folgenden Gruppe von Behandlungen ausgewählt ist: Vasodilatatoren, Diuretika und pulmonale Vasodilatatoren, neurohormonelle Antagonisten, Betablocker und Inotropika.

## Revendications

1. Système médical (41, 42) permettant de déterminer un schéma thérapeutique destiné à un patient présentant un état, le système (41, 42) comprenant :
au moins un capteur (10, 11) configuré pour détecter la pression auriculaire droite et la pression auriculaire gauche ; et
un récepteur configuré pour recevoir des données de mesure correspondant à la pression auriculaire droite détectée et à la pression auriculaire gauche détectée, pour recevoir l'état du patient, et transmettre à un dispositif d'affichage les données de mesure reçues ;
une unité de mémoire configurée pour stocker les données de mesure reçues et l'état du patient ; et
un processeur configuré pour :
comparer la pression auriculaire droite détectée à une pression auriculaire droite de base de manière à déterminer une première comparaison ;
comparer la pression auriculaire gauche détectée à une pression auriculaire gauche de base de manière à déterminer une seconde comparaison ; et
déterminer le schéma thérapeutique destiné au patient sur la base des comparaisons et de l'état du patient,
ledit état étant au moins un état choisi dans le groupe de : une insuffisance cardiaque gauche, une insuffisance cardiaque droite et un trouble pulmonaire primitif.

2. Système médical (41, 42) selon la revendication 1, afin de déterminer le schéma thérapeutique du patient ledit processeur étant configuré pour : comparer la pression auriculaire droite détectée et la pression auriculaire gauche détectée.

3. Système médical (41, 42) selon l'une quelconque des revendications 1 ou 2, afin de déterminer le schéma thérapeutique destiné au patient ledit processeur étant configuré pour fournir une notification destinée à augmenter la dose du schéma thérapeutique.

4. Système médical (41, 42) selon l'une quelconque des revendications 1 ou 2, afin de déterminer le schéma thérapeutique destiné au patient ledit processeur étant configuré pour fournir une notification destinée à diminuer la dose du schéma thérapeutique.

5. Système médical (41, 42) selon l'une quelconque des revendications 1 à 4, ledit processeur étant incorporé dans un dispositif médical implantable.

6. Système médical (41, 42) selon l'une quelconque des revendications 1 à 4, ledit processeur étant incorporé dans un dispositif situé à l'extérieur du patient.

7. Système médical (41, 42) selon l'une quelconque des revendications 1 à 6, afin de déterminer le schéma thérapeutique du patient ledit processeur étant configuré pour fournir une notification destinée à augmenter au moins un traitement choisi dans le groupe de traitements suivant : les vasodilatateurs, les diurétiques, les vasodilatateurs pulmonaires, les antagonistes neuro-hormonaux, les bêtabloquants et les inotropes.

8. Système médical (41, 42) selon l'une quelconque des revendications 1 à 6, afin de déterminer le schéma thérapeutique du patient ledit processeur étant configuré pour fournir une notification destinée à diminuer au moins un traitement choisi dans le groupe de traitements suivant : les vasodilatateurs, les diurétiques, les vasodilatateurs pulmonaires, les antagonistes neuro-hormonaux, les bêtabloquants et les inotropes.

9. Procédé mis en oeuvre par ordinateur (200) permettant de déterminer un schéma thérapeutique destiné à un patient présentant un état, le procédé comprenant :
la réception d'au moins une mesure correspondant à une pression auriculaire droite détectée pour le patient ;
la réception d'au moins une mesure correspondant à une pression auriculaire gauche détectée pour le patient ;
la réception de l'état du patient ;
la comparaison de la pression auriculaire droite détectée à une pression auriculaire droite de base de manière à déterminer une première comparaison ;
la comparaison de la pression auriculaire gauche détectée à une pression auriculaire gauche de base de manière à déterminer une seconde comparaison ;
la détermination du schéma thérapeutique destiné au patient sur la base de la première comparaison, de la seconde comparaison et de l'état du patient ; et
la transmission à un dispositif d'affichage : de ladite au moins une mesure correspondant à la pression auriculaire droite détectée, de ladite au moins une mesure correspondant à la pression auriculaire gauche détectée, et du schéma thérapeutique déterminé,
ledit état du patient étant au moins un état choisi dans le groupe de :
une insuffisance cardiaque gauche, une insuffisance cardiaque droite et un trouble pulmonaire primitif.

10. Procédé (200) selon la revendication 9, ladite détermination du schéma thérapeutique du patient comprenant la comparaison de la pression auriculaire droite détectée et de la pression auriculaire gauche détectée.

11. Procédé (200) selon l'une quelconque des revendications 9 ou 10, ladite détermination du schéma thérapeutique destiné au patient comprenant la fourniture d'une notification destinée à augmenter la dose du schéma thérapeutique.

12. Procédé (200) selon l'une quelconque des revendications 9 ou 10, ladite détermination du schéma thérapeutique destiné au patient comprenant la fourniture d'une notification destinée à diminuer la dose du schéma thérapeutique.

13. Procédé (200) selon l'une quelconque des revendications 9 à 12, ladite détermination du schéma thérapeutique du patient comprenant la fourniture d'une notification destinée à augmenter au moins un traitement choisi dans le groupe de traitements suivant : les vasodilatateurs, les diurétiques, les vasodilatateurs pulmonaires, les antagonistes neuro-hormonaux, les bêtabloquants et les inotropes.

14. Procédé (200) selon l'une quelconque des revendications 9 à 12, ladite détermination du schéma thérapeutique du patient comprenant la fourniture d'une notification destinée à diminuer au moins un traitement choisi dans le groupe de traitements suivant : les vasodilatateurs, les diurétiques et les vasodilatateurs pulmonaires, les antagonistes neuro-hormonaux, les bêtabloquants et les inotropes.
